(19) **Europäisches Patentamt**
**European Patent Office**
**Office européen des brevets**

(11) **EP 2 982 985 B1**

(12) **EUROPEAN PATENT SPECIFICATION**

(45) Date of publication and mention
of the grant of the patent:
**05.09.2018 Bulletin 2018/36**

(51) Int Cl.:
***C12Q 1/6886*** (2018.01)

(21) Application number: **14779566.0**

(86) International application number:
**PCT/KR2014/002958**

(22) Date of filing: **07.04.2014**

(87) International publication number:
**WO 2014/163444 (09.10.2014 Gazette 2014/41)**

(54) **SYSTEM FOR PREDICTING PROGNOSIS OF LOCALLY ADVANCED GASTRIC CANCER**

SYSTEM ZUR VORHERSAGE EINER PROGNOSE VON LOKAL FORTGESCHRITTENEM MAGENKREBS

SYSTÈME DE PRÉDICTION DU PRONOSTIC D'UN CANCER GASTRIQUE LOCALEMENT AVANCÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.04.2013 KR 20130037334**

(43) Date of publication of application:
**10.02.2016 Bulletin 2016/06**

(73) Proprietor: **Novomics Co., Ltd.**
**Seoul (KR)**

(72) Inventors:
• **HUH, Yong-min**
Seoul 137-930 (KR)
• **NOH, Sung Hoon**
Seoul 158-755 (KR)
• **CHEONG, Jae-Ho**
Seoul 135-280 (KR)
• **SUH, Jin Suck**
Seoul 137-910 (KR)
• **PARK, Eun Sung**
Seoul 138-891 (KR)

(74) Representative: **Schiweck Weinzierl Koch**
**Patentanwälte Partnerschaft mbB**
**Ganghoferstraße 68 B**
**80339 München (DE)**

(56) References cited:
WO-A1-03/065035    WO-A1-2009/045115
WO-A2-2008/063521  JP-A- 2010 539 973
JP-A- 2011 500 071  KR-A- 20120 065 959
US-A1- 2010 184 046

• **J. Y. CHO ET AL: "Gene Expression Signature-Based Prognostic Risk Score in Gastric Cancer", CLINICAL CANCER RESEARCH, vol. 17, no. 7, 29 March 2011 (2011-03-29), pages 1850-1857, XP055307553, US ISSN: 1078-0432, DOI: 10.1158/1078-0432.CCR-10-2180**
• **HIROSHI INOUE ET AL: "Prognostic Score of Gastric Cancer Determined by cDNA Microarray 1", CLINICAL CANCER RESEARCH, vol. 8, 1 November 2002 (2002-11-01), pages 3475-3479, XP055307731,**
• **K. E. LEE ET AL: "Prognostic Significance of p53, nm23, PCNA and c-erbB-2 in Gastric Cancer", JAPANESE JOURNAL OF CLINICAL ONCOLOGY, vol. 33, no. 4, 1 April 2003 (2003-04-01), pages 173-179, XP055307806, DOI: 10.1093/jjco/hyg039**
• **GUAN ZHEN YU ET AL: "Overexpression of Grb2/HER2 signaling in Chinese gastric cancer: their relationship with clinicopathological parameters and prognostic significance", JOURNAL OF CANCER RESEARCH AND CLINICAL ONCOLOGY, SPRINGER, BERLIN, DE, vol. 135, no. 10, 1 April 2009 (2009-04-01), pages 1331-1339, XP019740678, ISSN: 1432-1335, DOI: 10.1007/S00432-009-0574-8**
• **MASAKI MORI: "Clinicopathological and biological significance of CDC28 protein kinase regulatory subunit 2 overexpression in human gastric cancer", INTERNATIONAL JOURNAL OF ONCOLOGY, 25 May 2011 (2011-05-25), XP055307799, GR ISSN: 1019-6439, DOI: 10.3892/ijo.2011.1056**

- M Yu Shabelnik ET AL: "Differential expression of PKD1 and PKD2 in gastric cancer and analysis of PKD1 and PKD2 function in the model system", Experimental oncology, 1 December 2011 (2011-12-01), pages 206-211, XP055307797, Ukraine Retrieved from the Internet: URL:http://www.nbuv.gov.ua/old_jrn/Chem_Biol/eol/2011_4/206.pdf

**Description**

[Technical Field]

The present invention relates to a novel prognosis predicting system capable of predicting prognosis of locally advanced gastric cancer through a gene expression comparative analysis method.

[Background Art]

Gastric adeno-carcinoma is the second leading cause of death with 700,349 deaths in the year 2000 and the fourth most commonly diagnosed cancer in the world. It is considered a single heterogeneous disease with several epidemiologic and histo-pathologic characters. Treatment of gastric cancer is mainly based on clinical parameters like TNM (tumor, node, and metastasis) staging which decide whether patients should be treated by surgery alone or surgery plus chemotherapy. Unlike breast cancer and colon cancer, gastric cancer is clearly classified into stage 1 to stage 4 according to a TNM staging system. There is a great difference between stage 1 and stage 4, that is, a 5-year survival rate is equal to or greater than 90% in stage 1, and is equal to or less than 20% in stage 4. Therefore, it can be understood that the TNM staging system has very excellent prognosis predictability [Reference document, 7th edition of the AJCC cancer staging Manual: stomach. Ann Surg Oncol 2010; 17: 3077-3079]. Based on the staging system, gastric cancer can be generally divided into early gastric cancer, locally advanced gastric cancer, locally advanced invasive gastric cancer, metastatic gastric cancer and the like.

Even though surgery is the main treatment for operable gastric cancer, the recurrence rate is high in advanced cases. Multimodality treatment including chemotherapy and chemo-radiation has been introduced to prevent recurrence and to improve the prognosis of gastric cancer patients. However the optimal approach for individual patients is lacking as the clino-pathological heterogeneity of tumors and the different outcomes of patients in the same stage limit to predict responsibility of adjuvant chemotherapy even though these treatment options improved general clinical outcomes in patients.

The depth of tumor invasion and nodal involvement are the two main prognostic factors in gastric cancer. More than 50% of gastric cancer patients were accompanied by lymph node metastases at diagnosis and showed bad prognosis with less than 30% 5 year survival rate. So, accurate categorization of lymph node metastasis from gastric cancer patients is fundamentally critical to decide a treatment subsequent after radical gastrectomy. However, nodal status alone does not explain the heterogeneity of prognostic outcomes and the responsibility of chemotherapeutic agents after surgery. Even the patients having same pathological stages including same nodal stages does not show same prognostic outcomes. So the defining biological differences among tumors responsible for inherent clinical heterogeneity are the most important step for the development of new therapeutic strategies of gastric cancer.

Understanding of biological features influencing prognostic outcomes of gastric cancer patients is quite difficult since gastric cancer is a heterogeneous disease having epidemiological and histopathological differences. Although there are many different prognostic factors including gastric cancer subtypes such as a diffusion type and an enterotype, the prognostic outcome of gastric cancer is mainly influenced by the stage. However, heterogeneous prognostic outcomes are obtained even in the same stage, and most of these heterogeneities were not completely explained. Identification of genetic features influencing a prognostic outcome difference in the same stage is very important to select a treatment method for a patient. However, most genetic features that have been discovered were not clinically used due to low reproducibility and insufficient information that may be used to select a treatment method. There are other important factors that restrict introduction of such prognosis, and none of these prognoses is able to control the stage when the prognostic outcomes of gastric cancer patients are defined. Therefore, introduction of a prognosis predictive factor in patients of the same stage is necessarily required.

WO 2009/045115 A1 describes methods and compositions for determining the prognosis of cancer in a patient and the use of genetic marker for the prediction of the prognosis of cancer based on cell proliferation signatures. Further, it describes a method of predicting the likelihood of long-term survival of a cancer patient, a method of determining a treatment regime for a cancer patient, a method of preparing a personalized profile for a cancer patient as well as kits and devices for carrying out these methods.

Cho et al. (2011, Clinical Cancer Research, vol. 17, no. 7, pages 1850-1857) reported on developing a practical biomarker-based risk score that can predict relapse of gastric cancer after surgical treatment, wherein the scoring system is based on the expression of six genes that predict the likelihood of relapse after curative resection.

Inoue et al. (2002, Clinical Cancer Research, vol. 8, pages 3475-3479) also reported on developing a prognostic scoring system, however, by using a cDNA microarray. 78 genes were therefore selected and a coefficient was determined for each gene, wherein a prognostic score was calculated by summing-up the value for each gene.

Lee et al. (2003, Japanese Journal of Clinical Oncology, vol. 33, no. 4, pages 173-179) discussed the prognostic significance of p53, nm23, proliferating cell nuclear antigen (PCNA) and c-erbB-2 in gastric cancer.

KR 2012 0065959 A describes a marker for predicting a gastric cancer prognosis, a composition and a kit for predicting gastric cancer prognosis comprising an agent for measuring the expression level thereof, and a method for predicting gastric cancer prognosis using said marker.

[Disclosure]

[Technical Problem]

An object of the present invention is to provide a new prognosis predicting system that finds important biological features influencing clinical outcomes of gastric cancer patients of locally advanced gastric cancer, and particularly, in Stage N0 (N0 regional lymph node metastasis), and is based on a gene expression risk score (RS).

[Technical Solution]

In view of the above object, the present invention provides a method of predicting prognosis for a subject diagnosed with gastric cancer, the method including: a step of determining a degree of expression of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1 in a biological sample including cancer cells obtained from the subject; and a step of calculating a risk score (RS) and an RS percentage (RS (%)) of the biological sample based on the degree of expression of the RNA transcript determined in the above step and determining prognosis according to the RS (%).
Further is described a method of predicting prognosis for a subject diagnosed with gastric cancer, the method including: a step of measuring an expression level of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1 in a biological sample including cancer cells obtained from the subject; and a step of determining an increase in expression of the transcript as an increased likelihood of positive clinical outcomes.
It is further described that the method of predicting progress may be used to predict a clinical outcome after surgical resection of locally advanced gastric cancer of Stage T1N0, Stage T2N0, Stage T3N0 or Stage T4N0 in TNM staging classification.
The present invention also provides a computer readable recording medium having a program for executing prognosis prediction of gastric cancer causing a computer to execute the steps of, inputting a determined degree of expression of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1 in a nucleic acid sample obtained from a patient into the program; executing the program and then calculating a risk score (RS) and an RS percentage (RS (%)) of the sample based on the degree of expression of RNA determined in the above step by the following Equations 1 and 2, and classifying a patient as a high risk group patient when a set value range of the RS (%) is 50% or more, an intermediate risk group patient when a set value range of the RS (%) is 25% or more and less than 50%, or a low risk group patient when a set value range of the RS (%) is less than 25% for overall survival (OS) without cancer recurrence after surgical resection of gastric cancers:

[Equation 1]

$$RS = HR_1 * normLogTransValue_1 + HR_2 * normLogTransValue_2 + ... + HR_n * normLogTransValue_n$$

[Equation 2]

$$RS\ (\%) = 100 \times (RS\ of\ biological\ sample - RS\ minimum\ value\ of$$

$$population)/(RS\ maximum\ value\ of\ population - RS\ minimum\ value\ of\ population)$$

wherein,

$HR_n$ denotes a hazard ratio of an n-th RNA transcript, and when the $HR_n$ is less than 1, it is converted to $-1/HR_n$ and used,
normLogTransValue$_n$ is a value related to expression of the n-th RNA transcript, and this value is a value whose scale is changed based on a median value with respect to all values of corresponding genes, and
the population refers to a certain number of groups having locally advanced gastric cancer of Stage T1N0, Stage T2N0, Stage T3N0 or Stage T4N0 having no lymph node metastasis in TNM staging classification, and the certain number is any integer at which an RS maximum value and minimum value are calculable.

[Advantageous Effects]

According to the present invention, it is possible to predict clinical outcomes after surgical resection of gastric cancer using a method in which a prediction model is generated for overall survival with respect to a gastric cancer patient group of Stage N0 in the TNM stage, a degree of expression of RNA transcripts influencing statistically significant survival is determined, a risk scoring system is generated therefrom, and a prognosis indicating value is calculated.

Also, in the present invention, when a gene set system according to biological functions of genes is used, it is possible to analyze gene groups according to biological functions of gastric cancer itself.

[Description of Drawings]

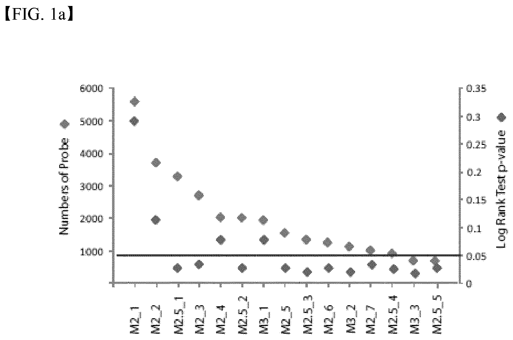

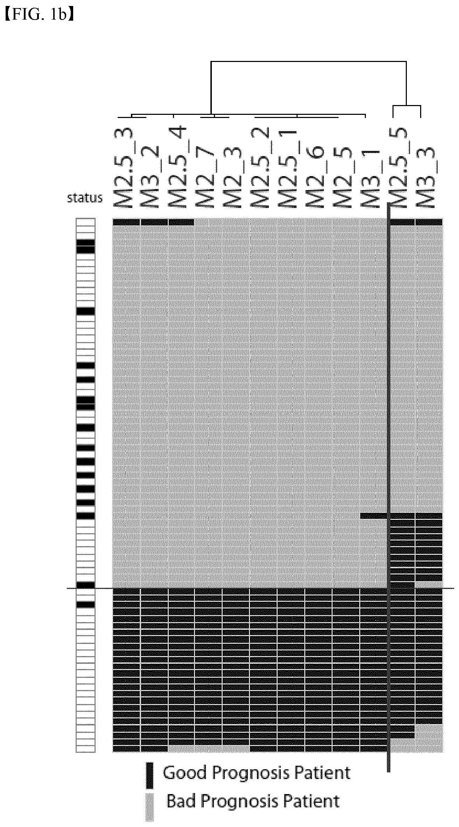

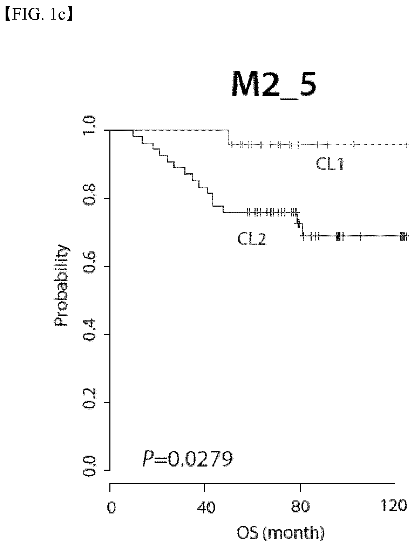

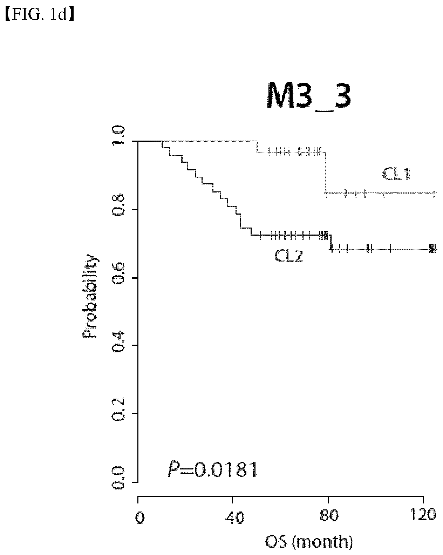

FIG. 1 shows prognostic outcomes of two main clusters generated by unsupervised hierarchical clustering analysis with the probes after variance filtration. FIG. 1a shows the number of probes used for unsupervised hierarchical clustering analysis after variance filtration and the prognostic p-values in Log Rank Test Analysis with two main classes generated by clustering analysis. Each of clusters were named based on the filtering criteria. The first number after M denote fold difference against mean values of each probes and second number denote the numbers of probes showing higher or lower expression comparing to the fold differences denoted by first number. For example, M2_3 is the cluster generated with the probes after variance filtration by selecting the probes having at least 3 samples showing more than 2 fold higher or lower expression against mean values. FIG. 1b shows a distribution of patient samples of two main clusters after variance filtration. Clustering analysis is performed on samples of two main classes after annotation of good prognosis group and bad prognosis group in log rank test. FIG. 1c shows a Kaplan Meier Plot of M2_5 cluster patients. p-value is obtained after log rank test. FIG. 1d shows a Kaplan Meier plot of M3_3 cluster patients. A P-value is obtained after the log rank test.

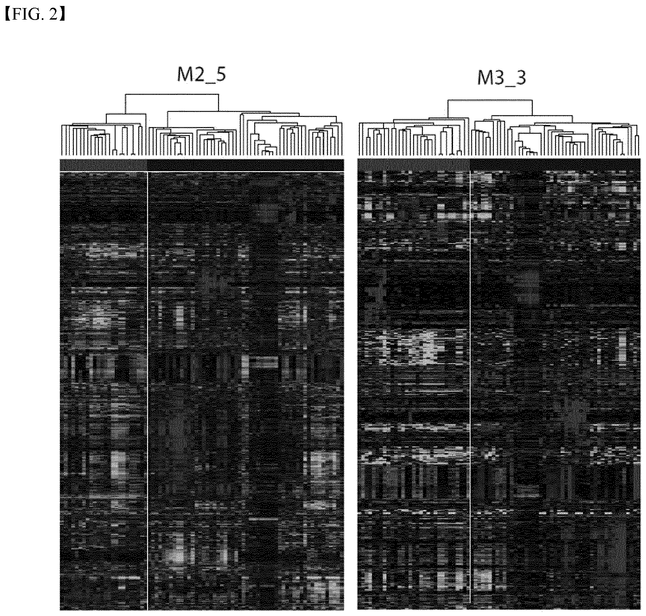

FIG. 2 shows two main representative clusters generated by unsupervised hierarchical clustering analysis after variance filtration. In clustering analysis of M2_5, probes having at least five samples showing an increase or a decrease of twice the average value or more are filtered and then the filtered 1556 probes are used. In clustering analysis of M3_3, probes having at least three samples showing an increase or a decrease of three times the average value or more are filtered and then the filtered 706 probes are used.

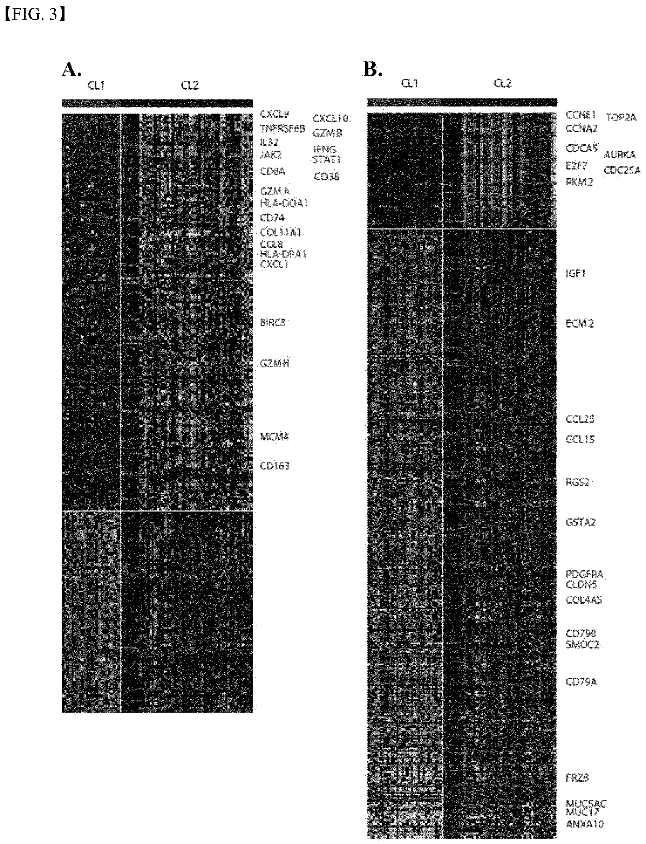

FIG. 3 shows the genes and biological features showing significant differences in the comparison of two main classes after unsupervised clustering analysis. FIG. 3A shows a heat map of supervised clustering with the probes showing statistical significance (p<0.001 and 2 fold difference, 554 probes) in the comparison of two main classes generated by clustering analysis of M2_5. FIG. 3B shows a heat map of supervised clustering with the probes showing statistical significance (p<0.001 and 2 fold difference, 453 probes) in the comparison of two main classes generated by clustering analysis of M3_3.

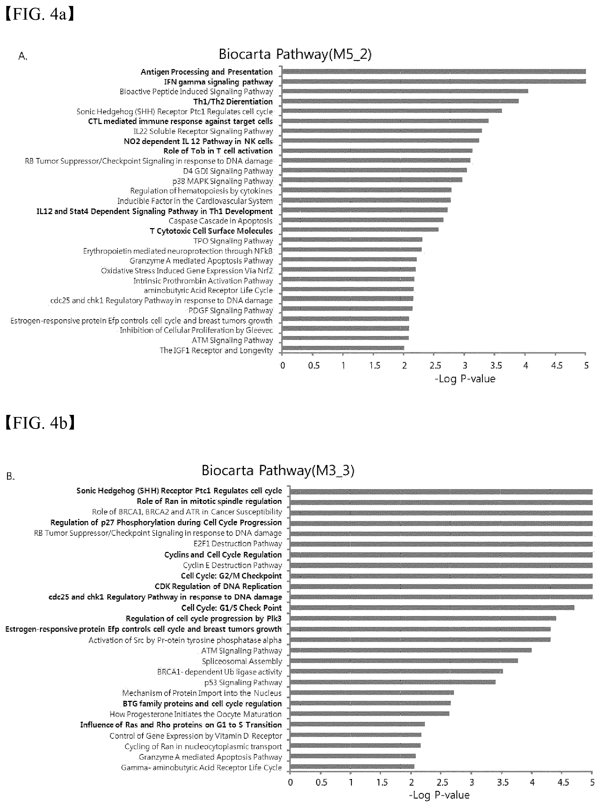

FIG. 4A shows GSEA results of two main classification groups of M2_5 in a Biocarta pathway database. FIG. 4B shows GSEA results of two main classification groups of M3_3 in a Biocarta pathway database.

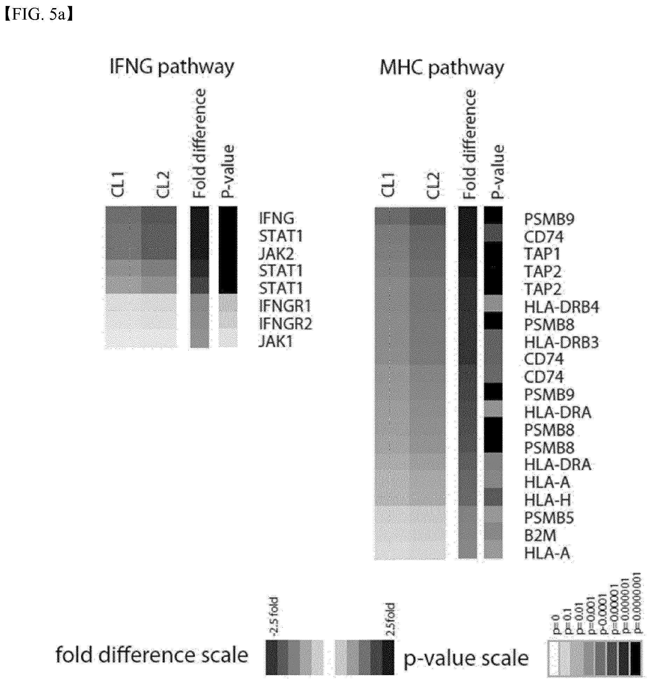

FIG. 5A-F shows expression of genes that show a significant increase in GSEA results (p<0.001). A heatmap shows an average expression level of each classification group of M2_5.

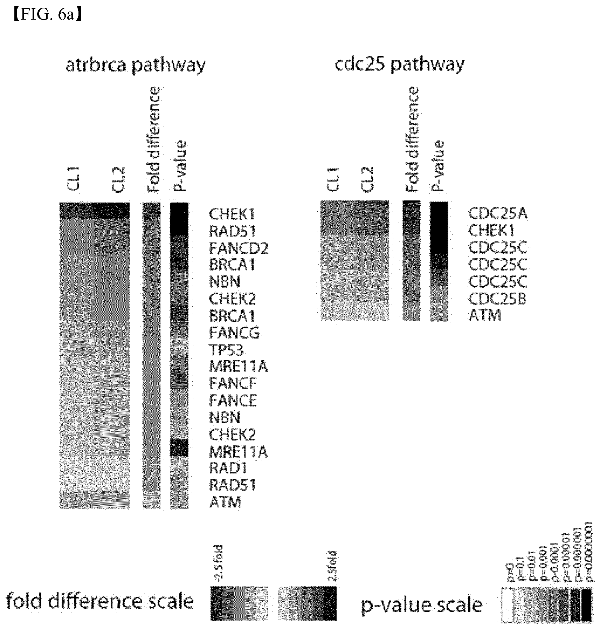

FIG. 6A-F shows expression of genes that show a significant increase in GSEA results (p<0.001). A heatmap shows an average expression level of each classification group of M3_3.

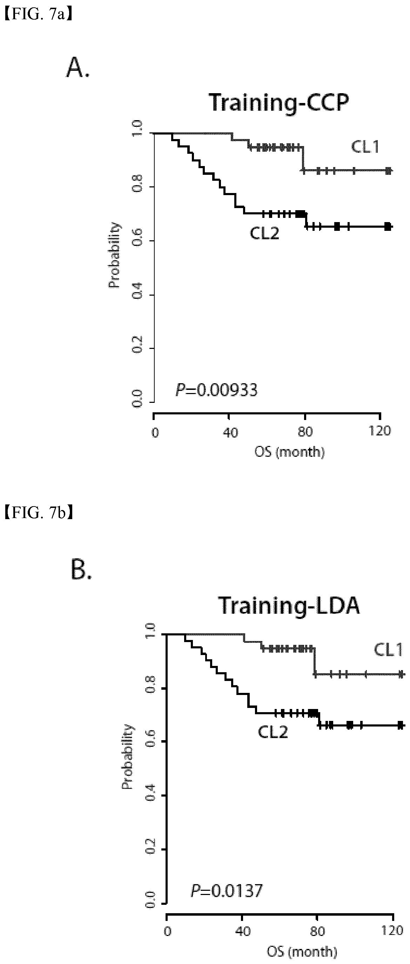

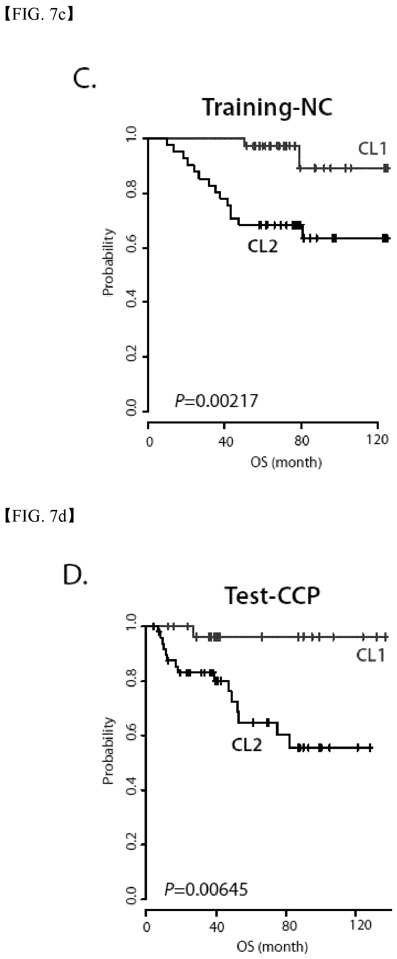

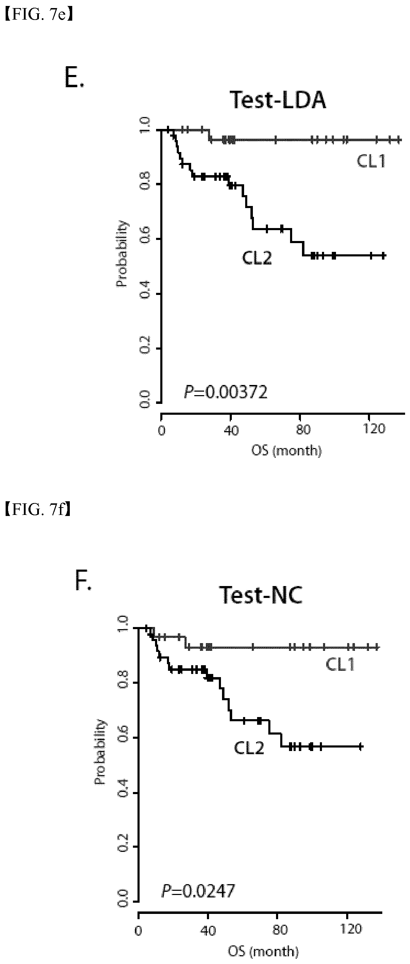

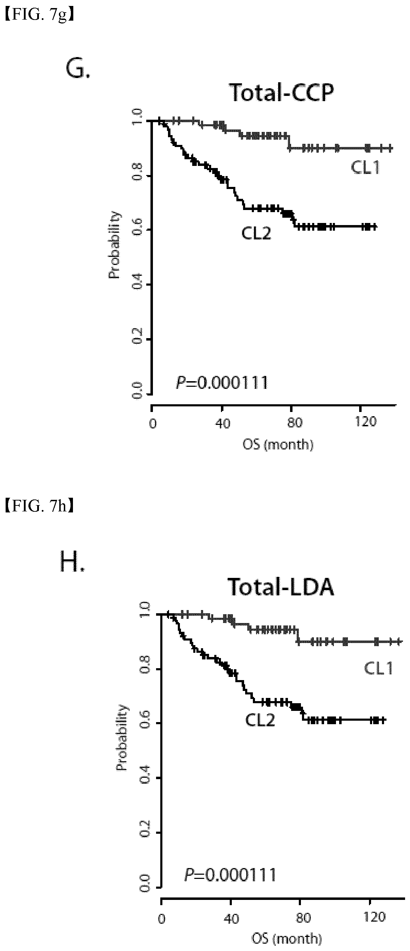

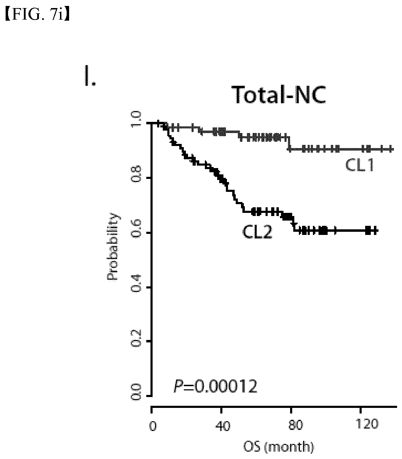

FIG. 7A-I shows prognosis prediction of N0 gastric cancer patients according to classification groups of M3_3. Probes are significantly different (p<0.001) when two classes defined by an M3_3 cluster are compared. Three different prediction algorithms (CCP, LDA, and NC) were used for this analysis. In order to estimate a prediction error of each model, leave-one-out cross-validation was used. A prognostic difference was estimated using the log rank test. FIGS. 7A to 7C show Kaplan Meier plots of predicted outcomes of training data (YUSH data). FIGS. 7D to 7F show Kaplan Meier plots of predicted outcomes of validation data (MDACC data). FIGS. 7G to 7I show Kaplan Meier plots of predicted outcomes of total sample data (YUSH data and MDACC data).

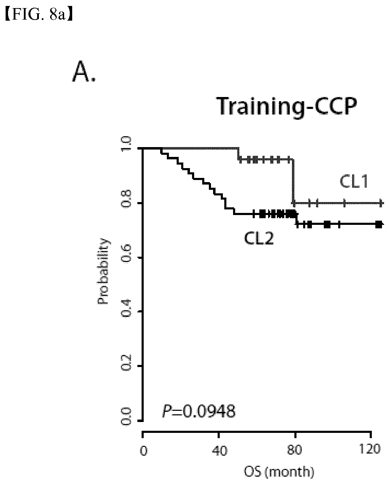

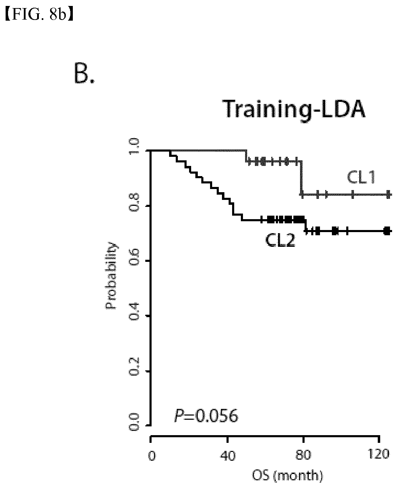

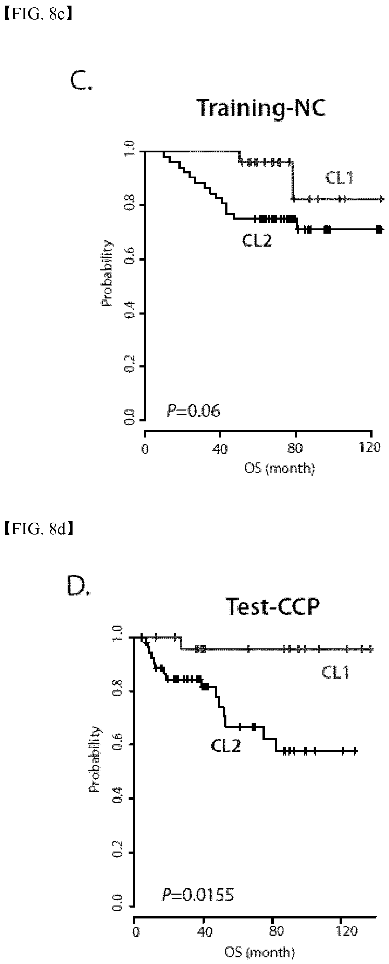

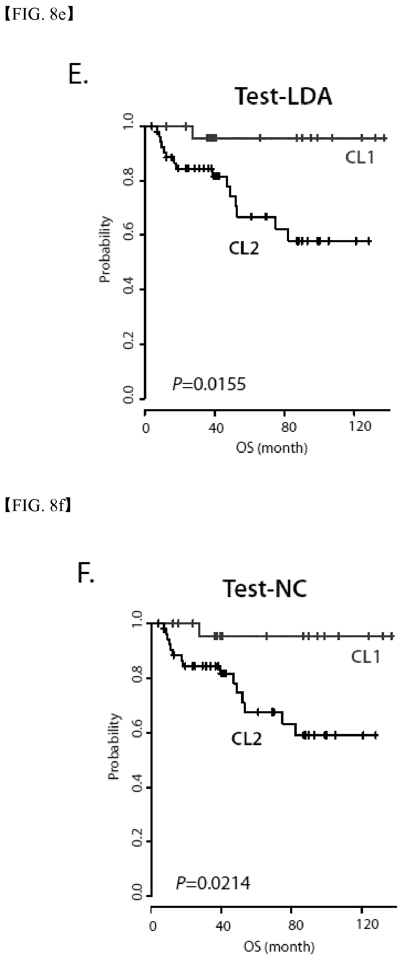

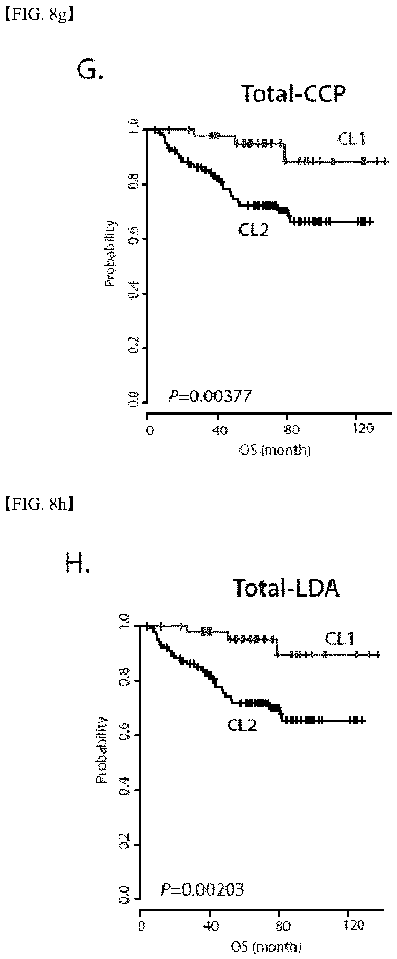

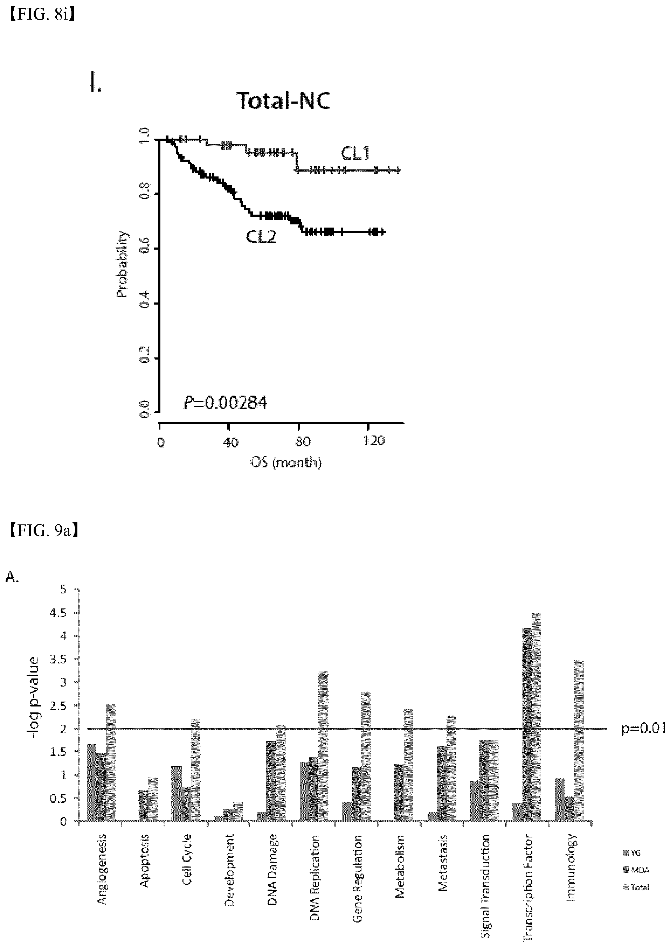

FIG. 8A-I shows prognosis prediction of N0 gastric cancer patients according to classification groups of M2_5. Probes are significantly different (p<0.001) when two classes defined by an M2_5 cluster are compared. Three different prediction algorithms (CCP, LDA, and NC) were used for this analysis. In order to estimate a prediction error of each model, leave-one-out cross-validation was used. A prognostic difference was estimated using the log rank test. FIGS. 8A to 8C show Kaplan Meier plots of predicted outcomes of training data (YUSH data). FIGS. 8D to 8F show Kaplan Meier plots of predicted outcomes of validation data (MDACC data). FIGS. 8G to 8I show Kaplan Meier plots of predicted outcomes of total sample data (YUSH data and MDACC data).

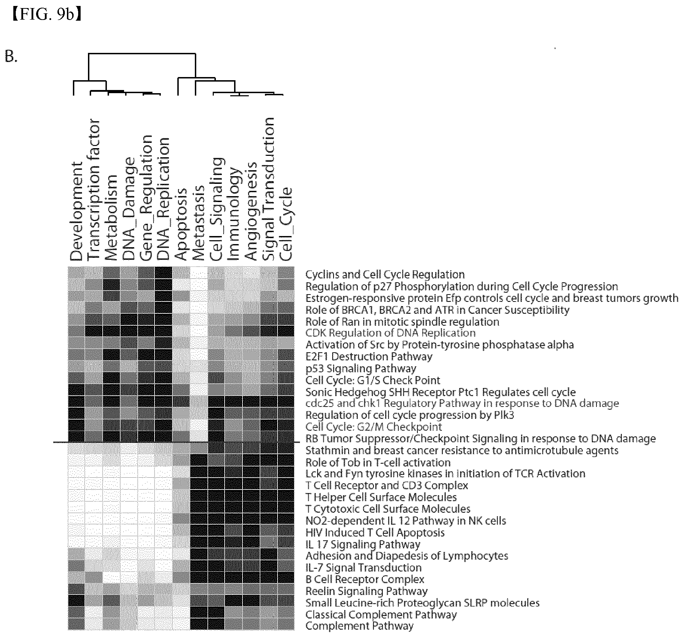

FIG. 9A-B shows an influence of functional gene categories predefined by CGAP in prognostic outcomes of N0 gastric cancer patients. FIG. 9A shows prognostic outcomes of the functionally categorized gene by CGAP in YUSH, MDACC and Total data sets. Unsupervised hierarchical clustering analysis was performed by using the genes in the functional gene categories of CGAP. The prognostic differences of main clusters were compared by Log Rank Test. The p-value of log rank test was converted into -log P-value and represented as bar graph. FIG. 9B shows biological features representing the differences of the main clusters in each functional gene categories. Gene set enrichment analysis (GSEA) were performed and the statistical significance of GSEA were represented as -log P-value.

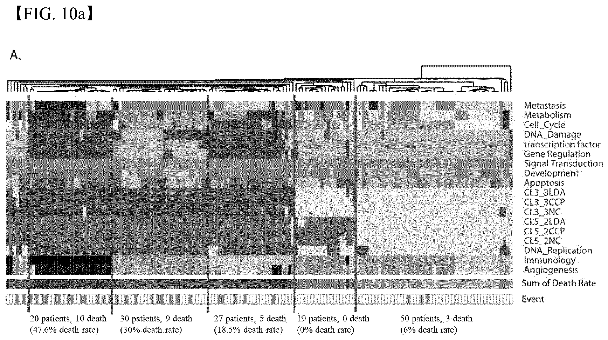

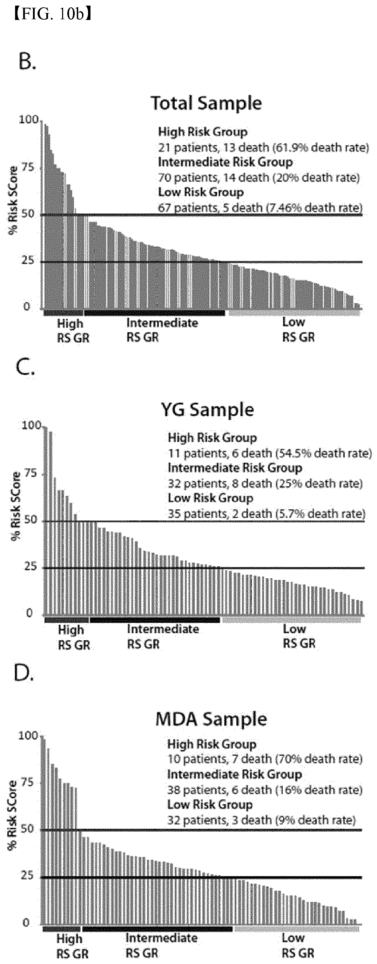

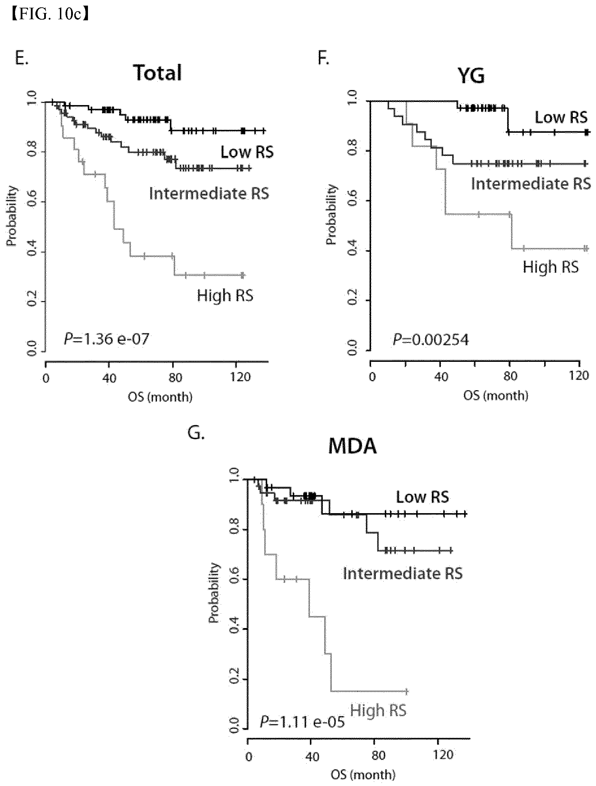

FIG. 10 shows generation of the percentage of a risk scoring system. FIG. 10A shows a heatmap of death rate defined by the prediction model and functional gene categories of CGAP. Every sample were annotated with the death rate of each class defined by classifier or clusters and unsupervised clustering analysis was performed to check patients distribution and influences of each categories of functional biology in the prognostic outcomes. FIG. 10B shows percentages of risk scores of each patient in total sample data set. FIG. 10C shows percentages of risk scores of each patient in YUSH sample data set. FIG. 10D shows percentages of risk scores of each patient in MDACC sample data set. FIGS. 10E to 10G show Kaplan Meier Plots of three different risk groups (High, Intermediate and Low Risk Group) defined by % risk score in three different data sets (YUSH, MDACC and Total sample data sets). The significance of prognostic differences between three different risk groups was defined by Log Rank Test.

[Modes of the Invention]

Hereinafter, a configuration of the present invention will be described in detail.

In order to define main biological features influencing a prognostic difference of gastric cancer patients of a relatively early stage having no lymph node metastasis, the present inventors generated whole genome wide gene expression profiles from patients having no lymph node metastasis. For this purpose, continuous variance filtration was performed while filtering criteria were changed and then an unsupervised hierarchical clustering analysis method was applied. Prognostic outcomes of two main classification groups defined according to clustering analysis were estimated using the log rank test. Since this analysis is self-analysis using genes representing all biological features of cancer patients, biological features representing different prognosis groups may be main biological features influencing a prognostic difference, and may be used as a potential target for development of treatment methods.

As an analysis result, two different biological features (cell proliferation and an immune response) mainly influencing a prognostic difference of gastric cancer patients of Stage N0 was identified. These two biological features were generally preserved regardless of classification groups based on variance filtration or functional gene classification of CGAP. Verification of such classification groups representing biological features of cell proliferation and immune activation was performed on an independent data set, and prognostic outcomes similar to that of a training data set were shown in the log rank test. A correct prediction ratio was examined using a leave-one-out cross-validation method, and the result was shown in a range of 85 to 96% according to a type of a classification group and a prognosis prediction algorithm. A result in which expression of cell-proliferation-related genes increased in a good prognosis group was unexpected, since most cancer cells have a higher proliferation rate than normal cells. However, cell proliferation of early gastric cancer is more rapid than gastric cancer of an advanced stage, and thus it is assumed that stem cell features are obtained and a metastasis potential is changed in cell features. Another factor that can explain good prognostic outcomes of the patients having high cell proliferation rate is the responsibility against chemotherapeutic agents. The patients having high expression of cell proliferation signature in the chemotherapeutic agents treated patients showed good response as expected. However, the prognostic outcomes of MDACC validation data sets did not support this idea as the patients without chemotherapeutic agents treatment also showed good prognostic outcomes when they had high expression of cell proliferation signatures like the patients having treatments with chemotherapeutic agents. So, the reason for the good prognostic outcomes having high proliferation signature expression is not just because of the susceptibility of chemotherapeutic agents but also because of the physiologic differences reflecting high cell proliferation biology.

The systematic finding of the influence of immune signature activation for the good prognosis of gastric cancer patients without lymph node invasion proved the critical roles of immune activation, especially for the activation status of CTLs in the cancer patient treatment. The significant role of immune response was previously reported in gastric cancer proving the high Foxp3 positive regulatory T-cell density in the sentinel lymph node is associated with downstream non-sentinel lymph node metastasis in gastric cancer.

The critical roles of the immune activation in the control of tumor progression were reported in several papers and it is generally accepted as another therapeutic option in many cancer types. Snail induced epithelial-mesenchymal transition accelerates induction of immune suppression and the mouse having the system to introduce exogeneous antigens into genetically engineered mouse lung cancers to mimic tumor neo-antigens showed endogeneous T cell response and delayed tumor progression. Active immunotherapy by vaccination with lethally irradiated, autologous tumor cells engineered to secret granulocyte-macrophage colony stimulating factor and antibody blockage of cytotoxic T lymphocyte-associated antigen-4 (CTLA-4) disrupted tumor vasculature by targeting tumor angiogenesis.

It is already known that tumors co-opt certain immune check point pathways as a major mechanism of immune resistance, particularly against T cells that are specific for tumor antigens. The ultimate amplitude and quality of the response of T cells is initiated through antigen recognition by T cell receptor and is regulated by a balance between co-stimulatory and inhibitory signals. The agonists of co-stimulatory receptors and antagonist of inhibitory signals both result in the amplification of antigen specific T cell responses and the blockage of immune checkpoints show the potentials of anti-tumor immune responses as human cancer therapeutics. Especially, CTLA-4 is important immune-checkpoint receptor, which down-modulates the amplitude of T cell activation. CTLA-4 antibodies were approved by US Food and Drug Administration (FDA) as immunotherapeutic agents and clinical studies using antagonistic CTLA4 antibodies demonstrated a survival benefit in patients with advanced melanoma. So the introduction of antagonistic CTLA-4 antibodies into gastric cancer patient treatment can be another treatment option for the patients having bad prognostic outcomes even in early N0 patients. The genetic signature from this study can guide to select right patients for that treatment option.

The present inventors proved that two distinct biological features mainly including cell-proliferation-related features and immune-response-related features are key biological features influencing prognostic outcomes of gastric cancer patients of Stage N0. Based on these findings, the present inventors propose that, when immunotherapy for gastric cancer patients is introduced and patients are selected for such a treatment, it should be based on genetic features in order to obtain maximum benefits for immunotherapy.

Therefore, the present invention provides a method of predicting prognosis for a subject diagnosed with gastric cancer, the method comprising: a step of determining a degree of expression of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1 in a biological sample including cancer cells obtained from the subject; and
a step of calculating a risk score (RS) and an RS percentage (RS (%)) of the biological sample based on the degree of expression of the RNA transcript determined in the above step and determining prognosis according to the RS (%).

In the method of predicting progress according to the present invention, among genes related to two main biological features influencing a clinical outcome of gastric cancer patients, immune activation and cell proliferation, in a gene group (functional categorized gene group) that is found in Cancer Genome Anatomy Project (CGAP) and functionally classified, genes having a statistical significance (p<0.001) in Cox regression analysis are selected as gene targets related to prognosis. A hazard ratio of the genes is multiplied by an expression value of the gene, and a risk score (RS) and an RS percentage (RS (%)) are obtained by the following Equations 1 and 2. A sample having the RS (%) of 50% or more is classified as a high risk group, a sample having the RS (%) of 25% or more and less than 50% is classified as an intermediate risk group and a sample having the RS (%) of less than 25% is classified as a low risk group for overall survival (OS). Therefore, it is possible to predict prognosis of a subject diagnosed with gastric cancer.

The RS and RS (%) may be calculated by the following Equations 1 and 2.

[Equation 1]

$$RS = HR_1 * normLogTransValue_1 + HR_2 * normLogTransValue_2 + ... + HR_n * normLogTransValue_n$$

[Equation 2]

$$RS\ (\%) = 100 \times (RS\ of\ biological\ sample - RS\ minimum\ value\ of\ population)/(RS\ maximum\ value\ of\ population - RS\ minimum\ value\ of\ population)$$

In the above equations,

$HR_n$ denotes a hazard ratio of an n-th RNA transcript, and when the $HR_n$ is less than 1, it is converted to $-1/HR_n$ and used,

$normLogTransValue_n$ is a value related to expression of the n-th RNA transcript, and this value is a value whose scale is changed based on a median value with respect to all values of the corresponding genes, and

the population refers to a certain number of groups having locally advanced gastric cancer of Stage T1N0, Stage T2N0, Stage T3N0 or Stage T4N0 having no lymph node metastasis in TNM staging classification, and the certain number is any integer at which an RS maximum value and minimum value are calculable.

The number of populations is not specifically limited. In one embodiment, 158 tissues of locally advanced gastric cancer of Stage T1N0, Stage T2N0, Stage T3N0 or Stage T4N0 having no lymph node metastasis in TNM staging classification were used as the population.

In the above equation, the term "hazard ratio (HR)" refers to a coefficient reflecting cancer progression, recurrence, or a contribution of a therapy response. The hazard ratio may be derived by various statistical techniques. The hazard ratio (HR) value may be determined in various statistical models, for example, may be determined in multivariate Cox proportional hazard regression analysis. In one embodiment, when the HR value is used for an RS formula, if the HR value is equal to or greater than 1, the HR value may be directly used, and if the HR value is less than 1, $-1/HR$ value may be used.

Also, in the above equation, the term "expression value of the RNA transcript" refers to a value related to expression of individual genes, that is, RNA transcripts. The value may be determined using various known statistical methods. For example, as the expression value, p-value measured by Cox regression analysis is converted into a log2 function value, quartile normalization is performed thereon, and then the value may be used. As the expression value used in Equation 1, a value whose scale is changed based on a median value with respect to all values of corresponding genes was used.

According to one embodiment, RS may be determined as follows.

RS=-GART×3.584 + PTN×3.631 - PCNA×2.7027 + GLI3×4.073 + SMARCD3×2.266 - SULT1A3×3.278 + ILK×2.251 - FUCA1×2.80899 + PKD1×2.827 - TOP2A×1.7668 + ABL1×2.784 - CKS2×1.9685 + FZD1×4.302 - TIAL1×4.2553 + SGCD×2.494 - PIGF×2.6525 - CCNB1×2.4272 - CSK ×3.2573 + CRYAB×1.524 + TPM1×2.975 - RFC4×2.817 + GUCY1B3×2.801 - TYMS×2.0617 - FEN1×2.3148 + GNAI1×2.758 + CSRP1×1.642 - UNG×2.695 + AXL×2.018 + MAP1×B1.705 + VCL×2.478 + ITGA5×1.642 - LIG1×2.841 - HPRT1×2.95 - GRB2×3.636 - HMMR×1.98 - MCM4×2.02 + SRF×2.287 + DMPK×1.925 - ACP5×2.551 - CD38×2.16 - PRIM1×3.003 - CCNF×2.024 + GLRB×2.138 - IFNAR2×3.717 + HSPA2×1.734 - CLN3×2.445 - BUB1×1.74 + CALM1×2.839 - CDC2×1.562 + ATF4×5.677 - RRM1×3.717.

The RS calculated according to Equation 1 may be represented as RS (%) according to Equation 2.

The value determined above is changed to a corresponding rank in the population. A sample having the RS (%) of 50% or more is classified as a high risk group, a sample having the RS (%) of 25% or more and less than 50% is classified as an intermediate risk group and a sample having the RS (%) of less than 25% is classified as a low risk group for overall survival (OS). The high risk group may be determined as having bad prognosis, and the low risk group may be determined as having good prognosis. That is, the sample having the RS (%) value of 50% or more refers to the high risk group that has low overall survival for a period of 3 years or more, 6 years or more, or 10 years or more. The low risk group having the RS (%) of less than 25% has high overall survival for a period of 3 years or more, 6 years or more, or 10 years or more. The term "good prognosis" may represent an increased likelihood of positive clinical outcomes, and the term "bad prognosis" may represent a decreased likelihood of positive clinical outcomes.

The method may be beneficial for predicting a clinical outcome after surgical resection in a gastric cancer patient group of Stage N0, for example, locally advanced gastric cancer of Stage T1N0, Stage T2N0, Stage T3N0 or Stage T4N0 in the TNM stage.

The genes used for the method of predicting progress of the present invention may be divided into gene sets involved in an immune response and cell proliferation. In the good prognosis group, expression statistically significantly increases.

Gene sets involved in the immune response: GART, PTN, SULT1A3, FUCA1, PKD1, ABL1, TIAL1, SGCD, PIGF, CSK, CRYAB, TPM1, GUCY1B3, GNAI1, CSRP1, AXL, MAP1B, VCL, ITGA5, LIG1, HPRT1, GRB2, HMMR, CD38, GLRB, IFNAR2, HSPA2, CLN3, BUB1, ATF4 and RRM1.

Gene sets involved in cell proliferation/DNA repair: PCNA, GLI3, SMARCD3, ILK, TOP2A, CKS2, FZD1, CCNB1, RFC4, TYMS, FEN1, UNG, MCM4, SRF, DMPK, ACP5, PRIM1, CCNF, CALM1 and CDC2.

The gene sets involved in the immune response relate mainly to antigen processing and presentation (MHC pathway) and an IFN gamma signaling pathway, and additionally, Th1/Th2 differentiation (TH1TH2 pathway), a CTL-mediated immune response (CTL pathway) of a target cell, an N02 dependent IL12 pathway (N02IL12 pathway) in NK cells, roles of Tob (TOB1 pathway) in T cell activation, an IL12 and Stat4 dependent signaling pathway (IL12 pathway) in Th1 development and cytotoxic T cell surface molecules (cytotoxic T pathway).

The gene sets involved in cell proliferation/DNA repair relate to roles of BRCA1, BRCA2 and ATR in cancer susceptibility (ATR BRCA pathway), a cdc25 and chkl regulation pathway in response to DNA damage (cdc25 pathway), cyclin and cell cycle regulation (cell cycle pathway), a cyclin E destruction pathway (FBW7 pathway), a cell cycle:G1/S checkpoint (G1 pathway), a cell cycle:G2/M checkpoint (G2 pathway), CDK regulation (MCM pathway), a p27 phosphorylation regulation during cell cycle progression (P27 pathway), a Sonic Hedgehog (SHH) receptor Ptc1 (PTC1 pathway) regulating cell cycle, an RB tumor suppressor/checkpoint signaling in response to DNA damage (RB pathway), and an E2F1 destruction pathway (SKP2 E2F pathway).

Further is described:

A method of predicting prognosis for a subject diagnosed with gastric cancer, the method including: a step of measuring an expression level of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1 in a biological sample including cancer cells obtained from the subject; and a step of determining an increase in expression of the transcript as an increased likelihood of positive clinical outcomes.

The method may be an array-based method.

The expression level may be normalized with respect to an expression level of at least one RNA transcript.

The clinical outcome may be expressed for overall survival (OS).

In the method, expression levels of all RNA transcripts are measured, an increase in expression is analyzed, an increased or decreased likelihood of positive clinical outcomes is determined, and thus prognosis may be predicted.

The method may be beneficial for predicting a clinical outcome after surgical resection of locally advanced gastric cancer of Stage T1N0, Stage T2N0, Stage T3N0 or Stage T4N0 having no lymph node metastasis in TNM staging classification.

The present invention provides a computer readable recording medium having a program for executing prognosis prediction of gastric cancer causing a computer to execute the steps of: acquiring the degrees of expression of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1 determined in a nucleic acid sample obtained from a patient; calculating a risk score (RS) and an RS percentage (RS (%)) of the sample based on the degree of expression of RNA determined in the above step by the following Equations 1 and 2, and classifying a patient as a high risk group patient when a set value range of the RS (%) is 50% or more, an intermediate risk group patient when a set value range of the RS (%) is 25% or more and less than 50%, or a low risk group patient when a set value range of the RS (%) is less than 25% for overall survival (OS) without cancer recurrence after surgical resection of gastric cancers:

[Equation 1]

$$RS = HR_1 * normLogTransValue_1 + HR_2 * normLogTransValue_2 + ... + HR_n * normLogTransValue_n$$

[Equation 2]

$$RS\ (\%) = 100 \times (RS\ of\ biological\ sample - RS\ minimum\ value\ of\ population) / (RS\ maximum\ value\ of\ population - RS\ minimum\ value\ of\ population)$$

wherein,

$HR_n$ denotes a hazard ratio of an n-th RNA transcript, and when the $HR_n$ is less than 1, it is converted to $-1/HR_n$ and used,

normLogTransValue$_n$ is a value related to expression of the n-th RNA transcript, and this value is a value whose scale is changed based on a median value with respect to all values of corresponding genes, and

the population refers to a certain number of groups having locally advanced gastric cancer of Stage T1N0, Stage T2N0, Stage T3N0 or Stage T4N0 having no lymph node metastasis in TNM staging classification, and the certain number is any integer at which an RS maximum value and minimum value are calculable.

The recording medium may provide a medium beneficial for predicting a clinical outcome after surgical resection of locally advanced gastric cancer of Stage T1N0, Stage T2N0, Stage T3N0 or Stage T4N0 having no lymph node metastasis in TNM staging classification.

The RS and RS (%) are calculated by Equations 1 and 2.

In the recording medium, when a set value range of the RS (%) is 50% or more, it may be determined as a high risk group, when a set value range of the RS (%) is 25% or more and less than 50%, it may be determined as an intermediate risk group and when a set value range of the RS (%) is less than 25%, it may be determined as a low risk group for overall survival (OS). That is, the sample having the RS (%) value of 50% or more, the high risk group, is determined as having low overall survival for a period of 3 years or more, 6 years or more or 10 years or more. The low risk group having the RS (%) value of less than 25% is determined as having high overall survival for a period of 3 years or more, 6 years or more or 10 years or more. The term "good prognosis" may represent an increased likelihood of positive clinical outcomes, and the term "bad prognosis" may represent a decreased likelihood of positive clinical outcomes.

Unless otherwise defined, technical and scientific terms used herein have meanings that are generally understood by those skilled in the art. Terms will be defined below for the present invention.

The term "microarray" refers to a regular arrangement of hybridizable array components on a substrate, preferably polynucleotide probes.

The term "polynucleotide" refers in general to any polyribonucleotide or polydeoxyribonucleotide, for example, modified or non-modified RNA or DNA. In this specification, the term "polynucleotide" specifically includes cDNA.

The term "oligonucleotide" refers to a relatively short polynucleotide including a single-stranded deoxyribonucleotide, a single or double-stranded ribonucleotide, an RNA:DNA hybrid and double-stranded DNA without limitations. Oligonucleotides, for example, a single-stranded DNA probe oligonucleotide, are often synthesized by a chemical method in which, for example, a commercially available automated oligonucleotide synthesizer is used. However, the oligonucleotide may be prepared by various methods including an in vitro recombinant DNA-mediated technique and DNA expression in cells and organisms.

The term "differentially expressed gene" or "differential gene expression" refers to a gene that is activated at a higher or lower level in subjects with cancer such as gastric cancer than that in expression of normal or silent subjects. Also, genes activated at a higher or lower level in different stages of the same disease are included. The differentially expressed gene may be a gene that is activated or suppressed at a nucleic acid level or a protein level, or causes a different polypeptide product due to different splicing. Such a difference can be confirmed according to a change in, for example, an mRNA level of a polypeptide, surface expression, secretion or other distribution. In the present invention, when a difference between given gene expressions of normal subjects and subjects with a disease or various stages of subjects with a disease is about 1.5 times or more, about 4 times or more, about 6 times or more, or about 10 times or more, "differential gene expression" is considered to be exhibited.

The term "normalized" related to a gene transcript or a gene expression product refers to a level of a transcript or a gene expression product with respect to an average level of a transcript/product of a reference gene set. Here, reference genes ("housekeeping genes") are selected based on a minimum variation thereof in patients, tissues or treatments, or reference genes are all tested genes. The latter case is referred to in general as "global normalization," and a relatively great number of tested genes in total is important, preferably, greater than 50. Specifically, the term "normalized" related to an RNA transcript refers to a transcription level with respect to an average of transcription levels of a reference gene set.

The terms "expression threshold value" and "defined expression threshold value" are interchangeably used and refer to a level of a gene or a gene product. At a level above the threshold value, the gene or the gene product is used as a predictive marker of a patient response. The threshold value is representatively and experimentally defined based on clinical studies. The expression threshold value may be selected as maximum sensitivity, maximum selectivity (for example, only responders of one drug should be selected), or a minimum error.

The term "gene amplification" refers to a process in which a plurality of replication products of genes or gene fragments is generated in specific cells or cell lines. A replicated region (elongation of amplified DNA) is often referred to as an "amplicon". Often, an amount of produced mRNA, that is, a degree of gene expression, also increases in proportion to the number of generated replication products of specific genes.

In this specification, the term "prognosis" is used to predict a likelihood of death from cancer or the progress (including recurrence, metastatic spread, and drug resistance) of neoplastic diseases such as gastric cancer herein. The term "prediction" is used herein to describe a likelihood of survival of a patient for a specific period without cancer recurrence after surgical resection of a major tumor. Such prediction may be clinically used to select a treatment method that is the most appropriate for any specific patient and determine the treatment method. Such prediction serves as a valuable indicator for predicting whether a patient is likely to beneficially respond to a therapeutic regimen, for example, a surgical procedure, or a patient is able to survive for a long time after completing surgery. The term, "prediction index" may be used together with "risk score".

Unless otherwise indicated, the present invention may be performed using techniques of the related arts of molecular biology (including recombinant techniques), microbiology, cell biology and biochemistry.

1. Gene expression profiling

Gene expression profiling methods include a polynucleotide hybridization analysis-based method, a polynucleotide sequencing-based method, and a proteomics-based method. Exemplary methods of quantifying mRNA expression include northern blotting, in situ hybridization, an RNAse protection assay, and a PCR-based method such as a reverse transcription polymerase chain reaction (RT-PCR). Also, antibodies capable of recognizing two specific strands including two strands of DNA, two strands of RNA, two strands of a DNA-RNA hybrid or two strands of DNA-protein may be used. Representative sequencing-based gene expression analysis includes serial analysis of gene expression (SAGE) and gene expression analysis according to massively parallel signature sequencing (MPSS).

2. Microarray

In fresh or paraffin-embedded tumor tissues, an expression profile of cancer-related genes may be measured. In this method, sequences of interest (including cDNA and oligonucleotides) are plated or arranged on a microchip substrate. Then, the arranged sequences are hybridized with specific DNA probes of cells or tissues of interest. Similarly to the RT-PCR method, a supply source of mRNA is the total RNA isolated typically from a human tumor or tumor cell lines, and corresponding normal tissues or cell lines. Therefore, RNA may be isolated from various major tumors or tumor cell lines. In a microarray technique, PCR amplified insertions of cDNA clones are provided on a substrate in a dense array manner. Preferably, 10,000 or more nucleotide sequences are applied to the substrate. Micro-arranged genes immobilized on a microchip with respect to 10,000 elements are appropriate for hybridization under strict conditions. Fluorescently labeled cDNA probes may be generated through reverse transcription of RNA extracted from tissues of interest and mixing of fluorescent nucleotides. The labeled cDNA probe applied to the chip is hybridized to have specificity to each spot of DNA on the array. In order to remove non-specifically bound probes, washing is completely performed, and then the chip is scanned by a confocal laser microscope or other detecting methods, for example, a CCD camera. When hybridization of the arranged elements is quantified, it is possible to evaluate excess of corresponding mRNA. When a dual-color fluorescent dye is used, separately labeled cDNA probes generated from two RNA supply sources are hybridized on the array for each pair. Therefore, relative excess of transcripts from two supply sources corresponding to each specified gene is simultaneously determined. Through hybridization in a small scale, convenient and rapid evaluation of expression patterns of a great number of genes is provided. Such a method has selectivity necessary for detecting rare transcripts (these are expressed in a small number of replication products for each cell) and performing detection with at least about twice a difference of a degree of expression in a reproducible manner. Microarray analysis may be performed using commercially available devices according to the manufacturer's protocol, for example, an Affymetrix GenChip technique or Incyte's microarray technique.

3. General descriptions of mRNA isolation, purification and amplification

A technique of profiling gene expression using paraffin-embedded tissues has been described above. The best treatment choice(s) available for patients are determined based on a distinctive gene expression pattern identified in an observed tumor sample by analyzing finally obtained data.

An important object of the present invention is to provide prognosis information using measured expression of specific genes of gastric cancer tissues. In order to achieve such an object, it is necessary to compensate for (normalize) a difference in an amount of assayed RNA, a change in quality of used RNA, and other factors, for example, machine and worker differences. Therefore, in the assay, typically, a use of reference RNA including transcriptions from known housekeeping genes such as GAPD and ACTB is measured for mixing. Accurate methods of normalizing gene expression data are disclosed in the document ["User Bulletin #2" for the ABI PRISM 7700 Sequence Detection System (Applied Biosystems; 1997)]. Alternatively, normalization may be performed based on an average or a median signal (Ct) of assayed genes or a great number of all subsets thereof (global normalization approach). In research described in the following examples, a central standardization strategy was used, and in order to perform normalization, subsets of screened genes selected based on a low correlation with clinical performance were used.

The term "training set" refers to a subject sample in which a statistically significant RNA transcript for prognosis is extracted.

The term "validation set" or "test set" refers to a set for examining accuracy according to whether the extracted variable can actually determine good or bad prognosis. Such a method is used to determine efficacy in an independent sample in addition to an effective prognosis determining ability in a specific sample group.

4. Risk score of recurrence and applications thereof

Characteristics of computation in which a cancer prognosis predicting method regarding a probability of gastric cancer recurrence is classified include 1) a unique experiment mRNAs set (or a corresponding gene expression product) used to measure a recurrence probability, 2) a specific weight used when expression data is added to a formula, and 3) a threshold value used to divide patients into groups having different levels of risk, for example, low, intermediate and high risk groups. Through this computation, a numerical risk score (RS) and RS (%) are calculated.

In an experiment, a Lab assay is necessary for measuring a level of specified mRNA or an expression product thereof. However, fresh tissues, frozen tissues, or paraffin-embedded tumor biopsy specimens that have already been necessarily collected from patients, stored and immobilized may be used in very small amounts. Therefore, the experiment may be non-invasive and may be compatible with, for example, several different methods for tumor tissues collected through core biopsy or fine needle aspiration. According to this method, the risk score (RS) of cancer is determined as follows:

> (a) gene or protein expression profiles are created using a biological sample including cancer cells obtained from the subject;
> (b) a degree of expression of several individual genes, that is, an mRNA level, is quantified and an expression value of each gene is determined;
> (c) a subset of gene expression values including expression values of genes each connected by a cancer-related biological function and/or simultaneous expression is generated;
> (d) a degree of expression of each gene in one subset is multiplied by a coefficient reflecting relative contribution to a cancer recurrence reaction thereof in the subset, and the multiplied value is added to calculate a value of the subset;
> (e) the value of each subset is multiplied by a coefficient reflecting contribution to a cancer recurrence reaction thereof; and
> (f) a sum of values of each subset by which the coefficient is multiplied is obtained and the risk score (RS) and the RS (%) are obtained.

Here, contribution of each subset, which has no linear correlation with cancer recurrence, is included in only a predetermined threshold value or more. A negative value is assigned to a subset in which increased expression of a specified gene reduces a cancer recurrence risk. A positive value is assigned to a subset in which expression of a specified gene increases a cancer recurrence risk.

In specific embodiments, RS and RS (%) may be determined by

> (a) measuring a degree of expression of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1, and
> (b) calculating a risk score (RS) and an RS (%) by the following Equations 1 and 2.

[Equation 1]

$$RS = HR_1 * normLogTransValue_1 + HR_2 * normLogTransValue_2 + ... + HR_n * normLogTransValue_n$$

[Equation 2]

$$RS\ (\%) = 100 \times (RS\ of\ biological\ sample - RS\ minimum\ value\ of\ population)/(RS\ maximum\ value\ of\ population - RS\ minimum\ value\ of\ population)$$

In the above equations,

$HR_n$ denotes a hazard ratio of an n-th RNA transcript, and when the $HR_n$ is less than 1, it is converted to $-1/HR_n$ and used,
normLogTransValue$_n$ is a value related to expression of the n-th RNA transcript, and this value is a value whose scale is changed based on a median value with respect to all values of corresponding genes, and
the population refers to a certain number of groups having locally advanced gastric cancer of Stage T1N0, Stage T2N0, Stage T3N0 or Stage T4N0 having no lymph node metastasis in TNM staging classification, and the certain number is any integer at which an RS maximum value and minimum value are calculable.

[Examples]

Hereinafter, examples of the present invention will be described in detail. However, the following examples are only examples of the present invention, and the scope of the present invention is not limited to the following examples.

<Preparation example> Prognosis prediction subject selection and experiment design

In order to select prognosis prediction subjects, tumor samples and clinical data were obtained from gastric adenocarcinoma patients (YUSH, n=78) who had undergone gastrectomy as a primary treatment in Yonsei University Severance Hospital from 1999 to 2006. All samples were collected after receiving consent described in detail from patients. Research was approved by the Ethics Committee at Yonsei University Severance Hospital. Clinical data was obtained retrospectively. An overall survival period was determined as a time from surgery to death. Data was censored when a patient was alive for the last contact. YUSH data was used to characterize biological features mainly responsible for prognostic outcomes and to explore prognostic prediction model by using it as training data set.

In order to verify the prognosis prediction model and a risk scoring system, in the present invention, gene expression profiles created by MD Anderson Cancer Center were used. Tumor samples and clinical data were obtained from gastric adenocarcinoma patients who had undergone gastrectomy as a primary treatment in Yonsei University Severance Hospital, Korea University Guro hospital and Kosin University College of Medicine from 1999 to 2006. All samples were collected after receiving consent described in detail from patients. Research was approved by the Ethics Committee at MD Anderson Cancer Center.

(Gene expression data)

Yonsei University Severance Hospital performed an experiment and analysis of 78 samples in a YUSH data set. Gene expression profiles were created by hybridizing Illumina human bead arrays (HumanHT-12, v3.0, Illumina, San Diego, CA) including 48803 gene features and labeled cRNAs. Total RNAs were extracted from fresh frozen tissues using a mirVana™ RNA isolating and labeling kit (Ambion, Inc.). According to instructions of the manufacturer (Illumina), total RNAs of 500 ng were used for labeling and hybridization. A beadchip was scanned by an Illumina BeadArray Scanner, and then microarray data was normalized according to a quartile normalization method in a linear model for a microarray data (LIMMA) package in an R language environment (Bolstad BM, 2003). Primary microarray data may be used in an NCBI gene expression omnibus (GEO) public database (microarray platform GEO0000 and microarray data GEO0000). An experiment and analysis of 80 samples in an MDACC data set were performed by the Department of Systems Biology at MD Anderson Cancer Center, similarly to the process performed on the YUSH data set. Primary microarray data of the MDACC data set may be used in an NCBI gene expression omnibus (GEO) public database (microarray platform GEO0000 and microarray data GEO0000).

(Microarray data analysis)

Cluster analysis was performed by clusters and treeviews (http://rana.lbl.gov/EigenSoftware.htm). In order to perform cluster analysis, in data converted by a log base 2, a median value was described with respect to each gene expression value. In order to produce genes having different expression levels among patients, continuous gene filtration was performed while filtering criteria were changed. Unsupervised clustering analysis was performed after continuous variance filtration. A prognostic difference of two classes configured as two main clusters was examined according to the log rank test and the Kaplan Meier plot.

In order to analyze a microarray data set, BRB ArrayTools Version 4.1 (http://linus.nic.nih.gov./BRB-Array-Tools.html) was used. Before main data analysis and after quartile normalization, the data set was converted by a log base 2. In order to identify genes that were significantly differently expressed between two compared classes, a t-test of two samples was applied. In order to find features of a main biological function and a genetic pathway, gene set enrichment analysis (GSEA) was performed on 281 pathways listed in a Biocarta database.

In order to generate the prognosis prediction model, the YUSH data was used as a training set, and the MDACC data set was used as a validation set. In order to predict a class of an independent patient data set, three different prediction algorithm-based prediction modes (Linear Discriminant Analysis (LDA), Compound Covariate Predictor (CCP) and Nearest Centroid (NC)), which had already been developed, were applied. Similarly to evaluation of two sample tests, the model incorporated that were differentially expressed among genes at the 0.001 significance level, as assessed by two-sample test. In order to estimate a prediction error of each model, leave-one-out cross-validation (LOOCV) was used. For a leave-one-out cross-validation training set, all model-building processes including gene screening were repeated. Also, it was evaluated whether a cross-validated error rate estimate of significantly less than 1 could be expected from any prediction. In order to evaluate a prognosis force of the prediction model, a validation data set for the prediction model was used, and the result thereof was evaluated using the Kaplan Meier plot and the log rank test.

In order to evaluate a prognostic difference between classified patient groups, the Kaplan Meier plot and the log rank test were used.

In order to evaluate independent prognostic-factor-related survival, gene features, a tumor stage and pathological features as a covariance, multivariate Cox proportional hazard regression analysis was used.

(Development of prognostic risk scoring system)

To generate risk scoring system based on the genes mainly responsible for the tumorigenesis and metastasis, the prognostic influence of functionally categorized genes from the CGAP annotated genes were performed. The risk scoring system was generated using the genes with annotation in CGAP, and a significant prognosis value was obtained in Cox-regression analysis (p<0.001). The risk score was obtained by multiplying a median value of an expression value by a hazard ratio (HR), and summing the values. When the HR value was less than 1, it was converted to -1/HR. A percentage (percentile risk score) of the risk score was calculated by the following equation.

$$RS\ (\%)=100\times(RS\ of\ sample\text{-}RS\ minimum\ value\ of\ population)/(RS$$

$$maximum\ value\ of\ population\text{-}RS\ minimum\ value\ of\ population)$$

A sample having the RS (%) of 50% or more was classified as a high risk group. A sample having the RS (%) of 25% or more and less than 50% was classified as an intermediate risk group. Finally, a sample having the RS (%) of less than 25% was classified as a low risk group.

<Example 1> Examination of gene expression profile of N0 gastric cancer patients

According to continuous variance filtration performed while filtering criteria were changed, 15 clusters having two unique main clusters were generated. After variance filtration, a plurality of genes had different numbers of probes, 5612 to 701. In the log rank test, a p-value was different according to the variance filtration criteria, a maximum of 0.291 (M2_1: a cluster having 5612 probes after genes having at least one probe that showed an increase or a decrease of twice a median value or more were selected and variance filtration was performed thereon) to a minimum of 0.0181 (M3_3: a cluster having 706 probes after genes having at least three probes that showed an increase or a decrease of three times a median value or more were selected and variance filtration was performed thereon). In 11 clusters among 15 clusters, two main classes showing a statistically significant prognostic difference in the log rank test through unsupervised hierarchical clustering analysis were generated (FIG. 1a and Table 1).

[Table 1] The numbers of probes after variance filtration and p-values of log rank test of two main clusters generated by unsupervised hierarchical clustering analysis after variance filtration

| CLUSTER | Probe number | P-value |
|---|---|---|
| M2_1 | 5612 | 0.291 |
| M2_2 | 3724 | 0.114 |
| M2.5_1 | 3301 | 0.0279 |
| M2_3 | 2718 | 0.0346 |
| M2_4 | 2043 | 0.0782 |
| M2.5_2 | 2029 | 0.0279 |
| M3_1 | 1956 | 0.0782 |
| M2_5 | 1556 | 0.0279 |
| M2.5_3 | 1354 | 0.0208 |

(continued)

| CLUSTER | Probe number | P-value |
|---|---|---|
| M2_6 | 1266 | 0.0279 |
| M3_2 | 1143 | 0.0208 |
| M2_7 | 1026 | 0.0346 |
| M2.5_4 | 931 | 0.026 |
| M3_3 | 706 | 0.0181 |
| M2.5_5 | 701 | 0.0279 |

The number of probes after variance filtration and p-values of the log rank test of two main clusters obtained through unsupervised hierarchical clustering analysis after variance filtration.

11 clusters showing a statistical significance in the prognostic outcome were used and patient sample pattern analysis was performed thereon. As a result, configurations of two classes generated according to unsupervised clustering analysis showed a quite similar pattern regardless of filtering criteria. Two different patterns of a sample configuration were shown, and one or two samples of each class showed a difference in classification according to filtering criteria (FIG. 1b). Therefore, two clusters showing two different patterns of the sample configuration were selected (FIG. 2).

Only 1 patient died (a death rate of 4%) in the good prognosis group of M2_5 (a cluster having 1556 probes after genes having at least 5 probes that showed an increase or a decrease of twice a median value or more were selected and variance filtration was performed thereon), while 15 patients died (a death rate of 28%) in the bad prognosis group (log rank test p=0.0279, FIG. 1c). Only 2 patients died (a death rate of 6%) in the good prognosis group of M3_3, while 14 patients died (a death rate of 29.8%) in the bad prognosis group (log rank test p=0.0181, FIG. 1d).

<Example 2> Biological features of two main clusters

In order to define main genetic features of two classes showing such a difference in the prognostic outcomes, a t-test was performed on two samples. After unsupervised clustering analysis, when two classes showing two main clusters of M2_5 were compared, 2886 significantly different probes were generated (p<0.001).

FIG. 3A shows the heatmap of supervised clustering analysis using probes that show a twofold difference or more and have a statistical significance (p<0.001) when two classes of M2_5 were compared. Many of the genes related immune response (IFNG, GZMA, GZMB, CD8A, STAT1, JAK2, and HLADPA1) were highly increased its expression in good response group.

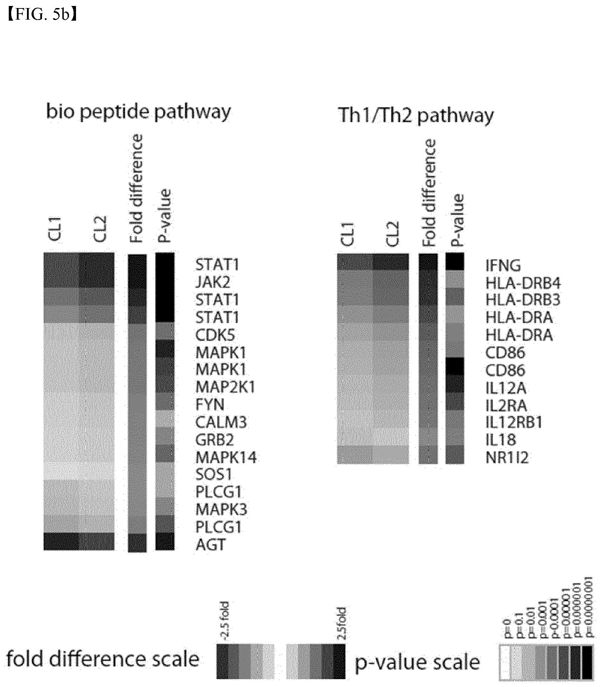

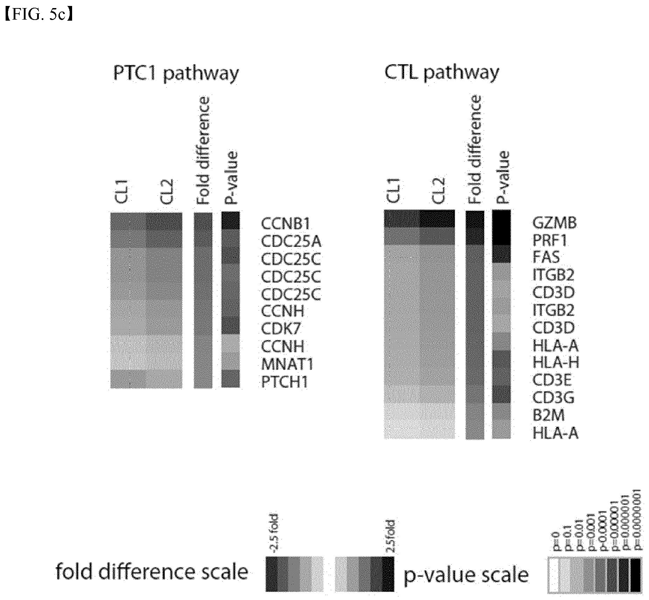

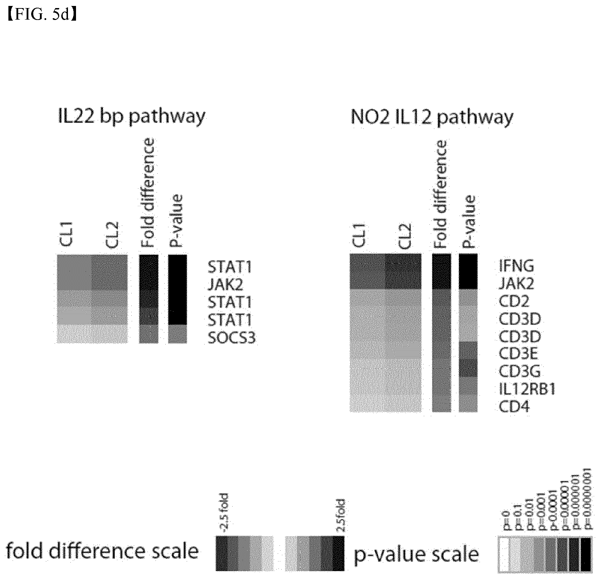

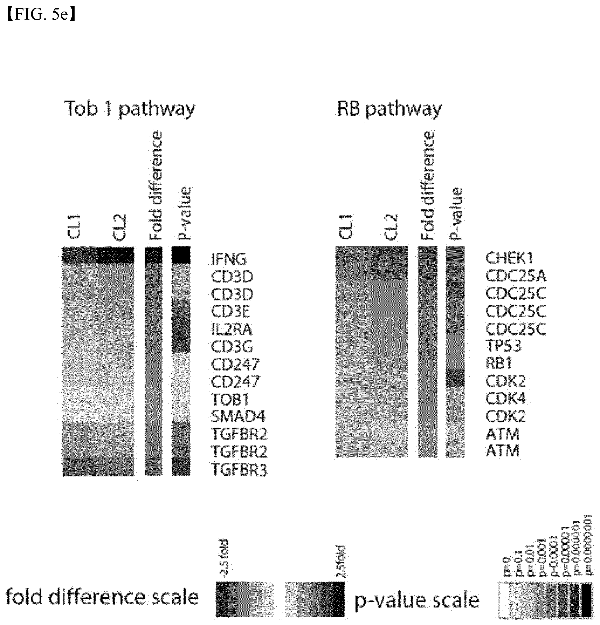

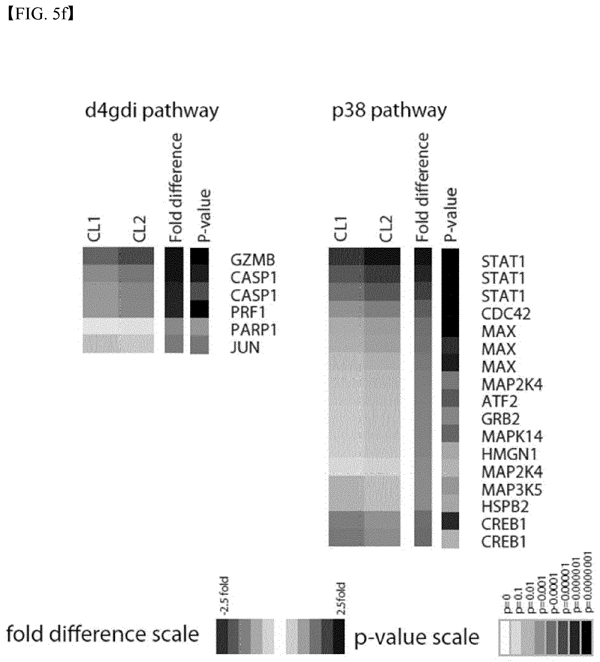

When GSEA of these two classes was performed in a Biocarta pathway database, the most significantly improved pathway was antigen processing and presentation (MHC pathway) and an IFN gamma signal transduction pathway (IFNG pathway) having a statistical significance (p=0.00001). Other than these two main signaling pathways, Th1/Th2 differentiation (TH1TH2 pathway), a CTL-mediated immune response (CTL pathway) of a target cell, an N02 dependent IL12 pathway (N02IL12 pathway) in NK cells, roles of Tob (TOB1 pathway) in T cell activation, an IL12 and Stat4 dependent signaling pathway (IL12 pathway) in Th1 development and cytotoxic T cell surface molecules (cytotoxic T pathway) are signaling pathways related to the immune response that significantly improved in GSEA in the Biocarta pathway database (FIG. 4A). Gene components of each significantly improved pathway showed unidirectional activation of genes related to immune activation in the good prognosis group (FIG. 5).

After unsupervised clustering analysis, when two classes showing two main clusters of M3_3 were compared, 2680 significantly different probes were generated (p<0.001).

FIG. 3B shows a heatmap of supervised clustering analysis using probes that show a difference of three times or more and have a statistical significance (p<0.001) when two classes of M3_3 are compared. Expression of genes related to cell proliferation (CCNE1, CCNA2, CDCA5, AURKA, E2F7, and CDC25A) and a gene (TOP2A) related to DNA repair significantly increased in a good response group.

When GSEA of these two classes was performed in a Biocarta pathway database, the most significantly improved pathway was roles of BRCA1, BRCA2 and ATR in cancer susceptibility (ATR BRCA pathway), a cdc25 and chkl regulation pathway in response to DNA damage (cdc25 pathway), cyclin and cell cycle regulation (cell cycle pathway), a cyclin E destruction pathway (FBW7 pathway), a cell cycle: G1/S checkpoint (G1 pathway), a cell cycle: G2/M checkpoint (G2 pathway), CDK regulation (MCM pathway), p27 phosphorylation regulation (P27 pathway) during cell cycle, a Sonic Hedgehog (SHH) receptor Ptc1 (PTC1 pathway) regulating cell cycle, an RB tumor repressor/checkpoint signaling (RB pathway) in response to DNA damage, and an E2F1 destruction pathway (SKP2 E2F pathway) (FIG. 4B, p=0.00001).

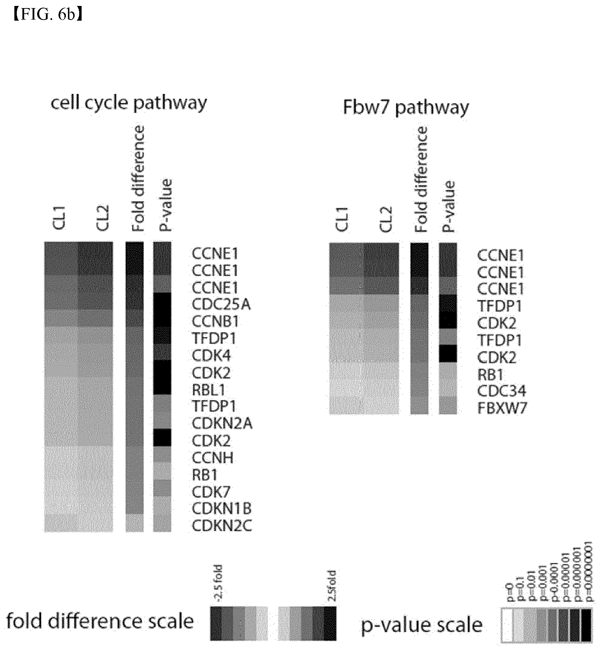

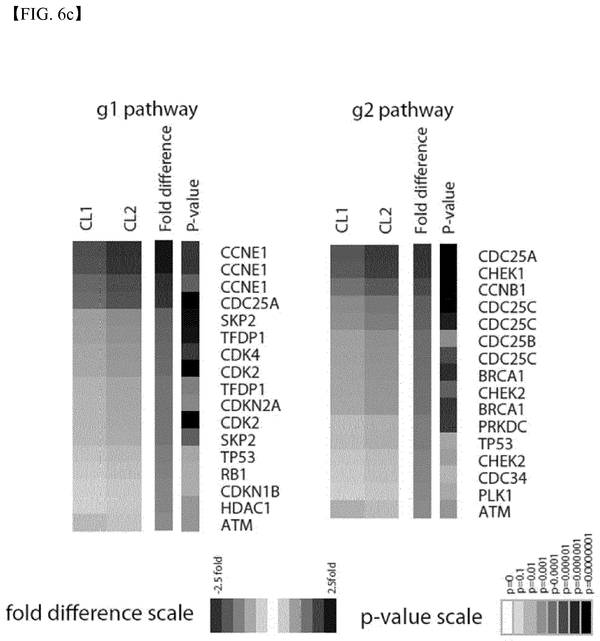

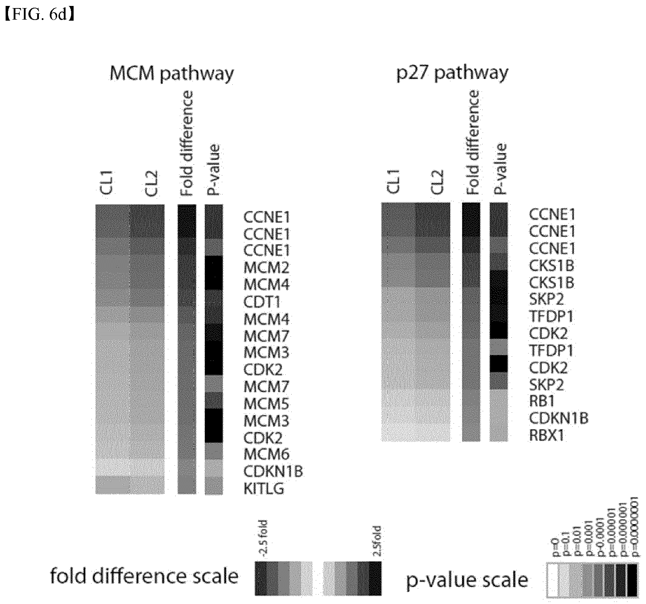

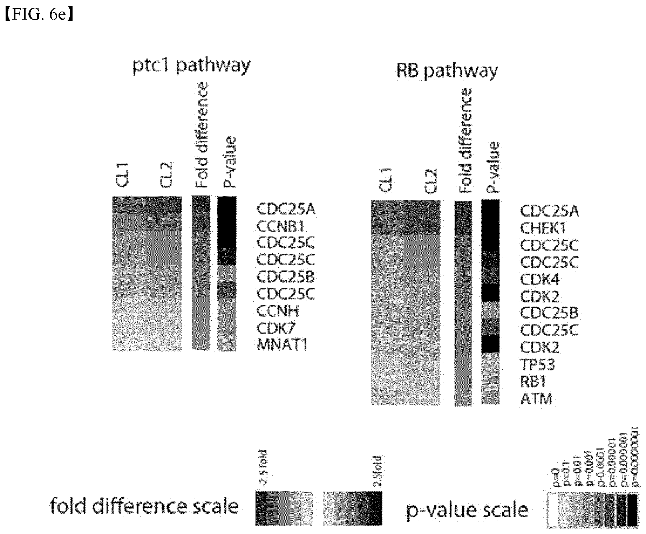

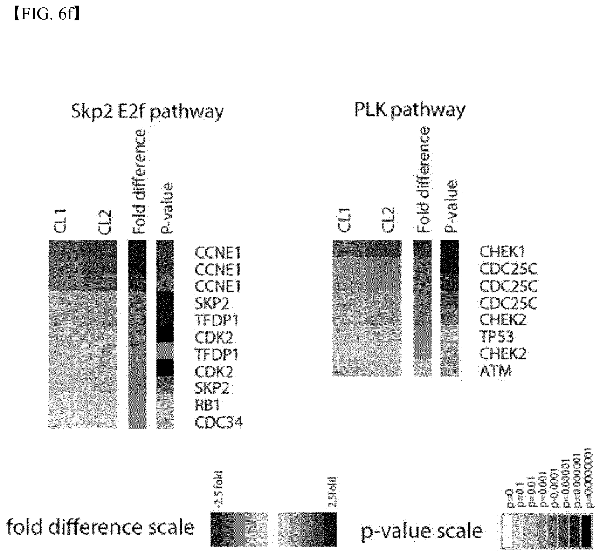

Gene components of each significantly improved pathway showed unidirectional activation of genes related to cell proliferation in the good prognosis group (FIG. 6).

<Example 3> Generation of prognosis prediction model

In order to generate the prognosis prediction model, three different prognosis prediction algorithms, that is, Compound Covariate Prediction (CCP), Linear Discriminant Analysis (LDA), and Nearest Centroid (NC), were used. In order to predict a classification group, significantly different genes between two classes at a significance level of 0.001 were used, and the leave-one-out cross-validation method was used to compute a correct prediction ratio.

In the training data set (YUSH data set) of classification groups of M3_3, a prognostic difference between two predicted groups was statistically significant (log rank test, CCP: p=0.00933, LDA: p=0.0137 and NC: p=0.00217), and a correct prediction ratio of classification groups of M3_3 was different from 85% to 92% (CCP: 86%, LDA: 85% and NC: 92%) (FIGS. 7A to 7C).

The MDACC data set was used to verify the classification group. The prediction result of MDACC test data set patients (80 patients) showed a pattern similar to that of the training YUSH data set in the prognostic outcome. The prognostic difference was statistically significant (log rank test, CCP: p=0.00645, LDA: p=0.00372 and NC: p=0.0247). The group classified as having good prognosis showed good prognostic outcomes such as a death rate of 3.3% (1 of 30 patients died) in CCP, a death rate of 3.2% (2 of 31 patients died) in LDA and a death rate of 6.45% (2 of 31 patients died) in NC. Also, groups classified as the bad prognosis group showed bad prognostic outcomes, a death rate of 30% (15 of 50 patients died) in CCP, a death rate of 30.6% (15 of 49 patients died) in LDA, and a death rate of 28.6% (14 of 49 patients died) in NC (FIGS. 7D to 7F).

In the predicted outcomes of total samples, a p-value of the log rank test was 0.000111 in CCP and LDA, and 0.000012 in NC. A very significant prognostic difference between two main classes was shown in all of the three different algorithms (FIGS. 7G to 7I).

Although the correct classification rate was much higher than in the classification groups of M3_3 (CCP 92%, LDA 90% and NC 95%), the predicted outcome in the test data set of M2_5 was not statistically significant (log rank test, CCP: p=0.0948, LDA: p=0.056 and NC: p=0.06) (FIGS. 8A to 8C).

The predicted outcome of MDACC test data set patients showed a pattern similar to that of the training YUSH data set in the prognostic outcome having a higher statistical significance. The prognostic difference was statistically significant (log rank test, CCP: p=0.0155, LDA: p=0.0155 and NC: p=0.0214), and the group classified as having good prognosis showed good prognostic outcomes such as a death rate of 3.8% (1 of 26 patients died) in CCP, LDA, and NC. Also, the group classified as the bad prognosis group showed bad prognostic outcomes such as a death rate of 27.8% (15 of 54 patients died) in CCP, LDA and NC (FIGS. 8D to 8F). In the predicted outcomes of total samples, a p-value of the log rank test was 0.00377 in CCP, 0.00203 in LDA, and 0.00284 in NC. A very significant prognostic outcome was shown in all of the three different algorithms (FIGS. 8G to 8i).

<Example 4> Functional gene categories from CGAP influencing prognosis of N0 gastric cancer patient

The gene annotation from CGAP at NIH is mainly featuring the functional gene categories influencing, tumorigenesis, tumor progression and metastasis of cancer. So the prognostic characterization based on these functional gene categories are quite informative as these approach reveals key biological features mainly responsible for the prognosis of certain stages of cancer. So we tested the influence of each gene categories from two data set from YUSH (n=78) and MDACC (n=80) and the combined total patients Data set (n=158).

The prognostic outcomes of main clusters generated by unsupervised hierarchical clustering analysis with functional gene categories varied between two different data sets. Angiogenesis is the only functional category in CGAP from YUSH data showing statistical significance (log rank test p=0.0215), while MDACC data set showed statistical significance in angiogenesis (p=0.0337), DNA Damage (p=0.0188), DNA Replication (p=0.0402), Metastasis (p=0.0235), Signal Transduction (p=0.0176) and Transcription factor (p=0.0000706). The combined patient data set revealed significance in most of functional gene categories except apoptosis and development (FIG. 9A).

GSEA of a Biocarta pathway database was performed on two classes defined by unsupervised hierarchical clustering analysis using probes of functional gene categories of total samples. As a result, functional gene categories showed two different patterns of significantly different gene set classification. Most gene sets (CDK regulation of DNA replication, an E2F1 destruction pathway, a cell cycle: G1/S checkpoint, a cell cycle: G2/M checkpoint, and a CDC25 and chkl regulation pathway in response to DNA damage) related to cell proliferation significantly increased when two classes including probes of functional gene categories of DNA replication, DNA damage, gene regulation, and metabolism and transcription factors were compared. Functional gene categories of metastasis, immunology, angiogenesis, cell signaling, signaling and a cell cycle showed the most significant difference in the immune response (roles of Tob in T cell activation, TCR activation, Lck and Fyn tyrosine kinases at the beginning of a T cell receptor and CD3 complex, helper T cell surface molecules, and an N02 dependent IL12 pathway in an NK cell and B cell receptor complex), and particularly, a gene set related to a T-cell-related immune response. It is reminding two main biological characters responsible for the biological features influencing prognostic differences of two classes generated with M2_5 and M3_3 clusters (FIG. 9B).

<Example 5> Generation of prognostic risk scoring system

After unsupervised clustering analysis, when patients were aligned according to a death rate of each class and the predicted outcomes of two classification groups M3_3 and M2_5, it was found that some patients were classified into a different class according to a classification group and a type of a cluster defined in functional gene categories. Therefore, classification and prognosis prediction based on features or a specific type of the classification group did not completely show the prognostic outcome even when the defined class showed a statistical significance in prognosis comparison of the log rank test (FIG. 10A). This is mainly caused by complex biological features of gastric cancer patients, and suggests that it is important to consider all aspects of biological or physiological features influencing a prognostic outcome of gastric cancer patients.

Therefore, next, in order to reflect main biological features causing a difference of the prognostic outcome of each functional gene categories, genes causing a prognostic difference in functional gene categories were examined. In Cox regression analysis, 51 genes having a statistical significance ($p<0.001$) were screened and used to generate a percentage of the prognostic risk scoring system (Table 2). A prognostic probe was selected according to Cox regression analysis ($p<0.001$) from probes with annotation in CGAP.

[Table 2]

| List of the probes used for the generation of percentile risk scoring system | | | | | |
|---|---|---|---|---|---|
| Unique id | Gene symbol | p-value | Hazard Ratio | UG cluster | CGAP |
| ILMN_ 1679476 | GART | 4.00E-07 | 0.279 | Hs.473648 | Gene Regulation; TRF |
| ILMN_ 1813753 | PTN | 1.00E-06 | 3.631 | Hs.371249 | Immunology |
| ILMN_ 1694177 | PCNA | 7.40E-06 | 0.37 | Hs.147433 | Cell Cycle; Gene Regulation; TRF |
| ILMN_ 1771962 | GLI3 | 1.34E-05 | 4.073 | Hs.21509 | Develoment |
| ILMN_ 2309180 | SMARCD3 | 1.77E-05 | 2.266 | Hs.647067 | Develoment |
| ILMN_ 1670517 | SULT1A3 | 2.56E-05 | 0.305 | Hs.460558 | Immunology |
| ILMN_ 2358980 | ILK | 3.31E-05 | 2.251 | Hs.706355 | Cell Cycle; Cell Signaling; Signal transduction |
| ILMN_ 1752728 | FUCA1 | 3.52E-05 | 0.356 | Hs.370858 | Immunology |
| ILMN_ 1669645 | PKD1 | 3.55E-05 | 2.827 | Hs.75813 | Cell Signaling; |
| ILMN_ 1686097 | TOP2A | 5.14E-05 | 0.566 | Hs.156346 | DNA Damage; DNA Replication |
| ILMN_ 1713732 | ABL1 | 5.25E-05 | 2.784 | Hs.431048 | Cell Signaling; Signal transduction |
| ILMN_ 2072296 | CKS2 | 5.78E-05 | 0.508 | Hs.83758 | Cell Cycle; Cell Signaling; Signal transduction |
| ILMN_ 2222065 | FZD1 | 5.85E-05 | 4.302 | Hs.94234 | Develoment |
| ILMN_ 1796855 | TIAL1 | 5.95E-05 | 0.235 | Hs.501203 | Gene Regulation; TRF |
| ILMN_ 1763457 | SGCD | 615E-05 | 2.494 | Hs.387207 | Immunology |

(continued)

| Unique id | Gene symbol | p-value | Hazard Ratio | UG cluster | CGAP |
|---|---|---|---|---|---|
| | List of the probes used for the generation of percentile risk scoring system | | | | |
| ILMN_1808938 | PIGF | 7.05E-05 | 0.377 | Hs.468415 | Immunology |
| ILMN_1712803 | CCNB1 | 7.57E-05 | 0.412 | Hs.23960 | Cell Cycle |
| ILMN_1754121 | CSK | 8.92E-05 | 0.307 | Hs.77793 | Signal transduction |
| ILMN_1729216 | CRYAB | 9.60E-05 | 1.524 | Hs.53454 | Immunology |
| ILMN_2278152 | TPM1 | 0.000104 | 2.975 | Hs.133892 | Immunology |
| ILMN_1724489 | RFC4 | 0.000117 | 0.355 | Hs.714318 | DNA Damage |
| ILMN_1782567 | GUCY1B3 | 0.000121 | 2.801 | Hs.77890 | Signal transduction |
| ILMN_1806040 | TYMS | 0.000187 | 0.485 | Hs.592338 | Metabolism |
| ILMN_2160929 | FEN1 | 0.000189 | 0.432 | Hs.409065 | DNA Replication |
| ILMN_1742044 | GNAI1 | 0.000194 | 2.758 | Hs.134587 | Cell Signaling; Signal transduction |
| ILMN_1811921 | CSRP1 | 0.000196 | 1.642 | Hs.108080 | Immunology |
| ILMN_1683120 | UNG | 0.000199 | 0.371 | Hs.191334 | DNA Damage |
| ILMN_1701877 | AXL | 0.000229 | 2.018 | Hs.590970 | Immunology |
| ILMN_2377900 | MAP1B | 0.000238 | 1.705 | Hs.335079 | Cell Signaling; Immunology; Metastasis |
| ILMN_1795429 | VCL | 0.000249 | 2.478 | Hs.643896 | Cell Signaling; Metastasis |
| ILMN_1792679 | ITGA5 | 0.00027 | 1.642 | Hs.505654 | Cell Signaling; Immunology; Metastasis |
| ILMN_1706779 | LIG1 | 0.000274 | 0.352 | Hs.1770 | DNA Damage; Immunology |
| ILMN_2056975 | HPRT1 | 0.000293 | 0.339 | Hs.412707 | Immunology |
| ILMN_1742521 | GRB2 | 0.000295 | 0.275 | Hs.444356 | Angiogenesis; Cell Signaling; Signal transduction |
| ILMN_2409220 | HMMR | 0.000304 | 0.505 | Hs.728200 | Cell Signaling; Metastasis |
| ILMN_1737205 | MCM4 | 0.000311 | 0.495 | Hs.460184 | Cell Cycle |
| ILMN_1803398 | SRF | 0.00035 | 2.287 | Hs.520140 | Cell Cycle; Cell Signaling; Develoment; Gene Regulation; TRF |
| ILMN_1689828 | DMPK | 0.00036 | 1.925 | | Cell Cycle; Cell Signaling; |
| ILMN_2078599 | ACP5 | 0.000365 | 0.392 | Hs.1211 | Cell Cycle; Cell Signaling; Immunology |
| ILMN_2233783 | CD38 | 0.000391 | 0.464 | Hs.479214 | Angiogenesis; Metastasis |

(continued)

| List of the probes used for the generation of percentile risk scoring system | | | | | |
|---|---|---|---|---|---|
| Unique id | Gene symbol | p-value | Hazard Ratio | UG cluster | CGAP |
| ILMN_ 1694502 | PRIM1 | 0.000392 | 0.333 | Hs.534339 | DNA Damage |
| ILMN_ 1773119 | CCNF | 0.000507 | 0.494 | Hs.1973 | Cell Cycle |
| ILMN_ 1669631 | GLRB | 0.000689 | 2.138 | Hs.32973 | Immunology |
| ILMN_ 1765146 | IFNAR2 | 0.00071 | 0.269 | Hs.708195 | Immunology |
| ILMN_ 2153916 | HSPA2 | 0.000723 | 1.734 | Hs.432648 | Immunology |
| ILMN_ 1669281 | CLN3 | 0.000802 | 0.409 | Hs.534667 | Immunology |
| ILMN_ 2202948 | BUB1 | 0.00084 | 0.575 | Hs.469649 | Immunology |
| ILMN_ 1778242 | CALM1 | 0.000851 | 2.839 | Hs.282410 | Cell Cycle; Cell Signaling; Signal transduction |
| ILMN_ 1747911 | CDC2 | 0.000889 | 0.64 | | Cell Cycle |
| ILMN_ 1672128 | ATF4 | 0.000969 | 5.677 | Hs.496487 | Develoment; TRF |
| ILMN_ 1771593 | RRM1 | 0.000994 | 0.269 | Hs.445705 | Immunology |

Among total patients (n=158), 21 patients were designated as a high risk group based on a percentage of the risk scoring system showing a risk score of 50% or more. A death rate of high risk group patients was very high at 61.9% in total samples, and patients of the two data sets showed quite similar clinical outcomes (YUSH: a death rate of 54.5%, and MDACC: a death rate of 70%).

70 patients were designated as an intermediate risk group (a risk score of 25% or more and less than 50%), and a death rate of the intermediate risk group was 20%. The clinical outcome of the YUSH data set was slightly worse than that of MDACC data patients showing a death rate of 25% with respect to YUSH patients, and MDACC data patients had a death rate of 16%. A total of 67 patients were designated as a low risk group, and a death rate of 7.45% was shown in total sample data. YUSH data patients had a death rate of 5.7%, which shows slightly better prognosis than MDACC data patients having a death rate of 9% (FIGS. 10B to 10D).

The prognostic difference was apparent and three different risk groups showed a very impressive statistical significance in the log rank test of total data sets such as a p-value of 1.36 e-07. In the YUSH data set, a p-value was 0.00254 in the log rank test. In the MDACC data set, a p-value was 1.11e-05 in the log rank test (FIGS. 10E to 10F).

It is further described that the present disclosure can be used as a diagnostic kit in the field of recurrence prognosis prediction of gastric cancer.

## Claims

1.   A method of predicting prognosis for a subject diagnosed with gastric cancer, the method comprising:

a step of determining a degree of expression of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 and RRM1 in a biological sample including cancer cells obtained from the subject; and
a step of calculating a risk score (RS) and an RS percentage (RS (%)) of the biological sample based on the degree of expression of the RNA transcript determined in the above step and determining prognosis according to the RS (%).

2. The method of claim 1,
   wherein the RS and RS (%) are calculated by the following Equations 1 and 2:

[Equation 1]

$$RS = HR_1 * normLogTransValue_1 + HR_2 * normLogTransValue_2 + ... + HR_n * normLogTransValue_n$$

[Equation 2]

$$RS\,(\%) = 100 \times (RS\ of\ biological\ sample - RS\ minimum\ value\ of\ population)/(RS\ maximum\ value\ of\ population - RS\ minimum\ value\ of\ population)$$

where,

HR$_n$ denotes a hazard ratio of an n-th RNA transcript, and when the HR$_n$ is less than 1, it is converted to -1/HR$_n$ and used,
normLogTransValue$_n$ is a value related to expression of the n-th RNA transcript, and this value is a value whose scale is changed based on a median value with respect to all values of corresponding genes, and
the population refers to a certain number of groups having locally advanced gastric cancer of Stage T1N0, Stage T2N0, Stage T3N0 or Stage T4N0 having no lymph node metastasis in TNM staging classification, and the certain number is any integer at which an RS maximum value and minimum value are calculable.

3. The method of claim 1,
   wherein the method is used to predict a clinical outcome after surgical resection of locally advanced gastric cancer of Stage T1N0, Stage T2N0, Stage T3N0 or Stage T4N0 having no lymph node metastasis in TNM staging classification.

4. The method of claim 1,
   wherein, in the method, when the RS (%) of the biological sample is 50% or more, it is determined as a high risk group, when the RS (%) of the biological sample is 25% or more and less than 50%, it is determined as an intermediate risk group, and when the RS (%) of the biological sample is less than 25%, it is determined as a low risk group for overall survival (OS).

5. A computer readable recording medium having a program for executing prognosis prediction of gastric cancer causing a computer to execute the steps of, acquiring the degrees of expression of RNA transcripts of GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CDC2, ATF4 and RRM1 determined in a nucleic acid sample obtained from a patient; calculating a risk score (RS) and an RS percentage (RS (%)) of the sample based on the degree of expression of RNA determined in the above step by the following Equations 1 and 2; and
   classifying a patient as a high risk group patient when a set value range of the RS (%) is 50% or more, an intermediate risk group patient when a set value range of the RS (%) is 25% or more and less than 50%, or a low risk group patient when a set value range of the RS (%) is less than 25% for overall survival (OS) without cancer recurrence after surgical resection of gastric cancers:

[Equation 1]

$$RS = HR_1 * normLogTransValue_1 + HR_2 * normLogTransValue_2 + ... + HR_n * normLogTransValue_n$$

[Equation 2]

RS (%)=100×(RS of biological sample-RS minimum value of population)/(RS maximum

value of population-RS minimum value of population)

wherein,

$HR_n$ denotes a hazard ratio of an n-th RNA transcript, and when the $HR_n$ is less than 1, it is converted to $-1/HR_n$ and used,

normLogTransValue$_n$ is a value related to expression of the n-th RNA transcript, and this value is a value whose scale is changed based on a median value with respect to all values of corresponding genes, and

the population refers to a certain number of groups having locally advanced gastric cancer of Stage T1N0, Stage T2N0, Stage T3N0 or Stage T4N0 having no lymph node metastasis in TNM staging classification, and the certain number is any integer at which an RS maximum value and minimum value are calculable.

**Patentansprüche**

1. Verfahren zur Vorhersage einer Prognose für eine Versuchsperson, bei der Magenkrebs diagnostiziert wurde, wobei das Verfahren Folgendes umfasst:

einen Schritt zur Bestimmung eines Expressionsgrades von RNA-Transkripten von GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 und RRM1 in einer biologischen Probe, die Krebszellen enthält und von der Versuchsperson gewonnen wurde; und

einen Schritt zur Berechnung einer Risikobewertung (RS) und eines RS-Prozentsatzes (RS (%)) der biologischen Probe auf der Grundlage des Expressionsgrades des RNA-Transkripts, der im obigen Schritt bestimmt wurde, und zur Bestimmung der Prognose gemäß RS (%).

2. Verfahren nach Anspruch 1,
wobei RS und RS (%) mittels folgender Gleichungen 1 und 2 berechnet werden:

[Gleichung 1]

$$RS = HR_1 * normLogTransValue_1 + HR_2 * normLogTransValue_2 + ... + HR_n * normLogTransValue_n$$

[Gleichung 2]

RS (%) = 100x(RS der biologischen Probe - RS-Minimalwert derPopulation)/(RS-Maximalwert der Population - RS Minimalwert der Population)

wobei

$HR_n$ einen Risikoquotienten eines n-ten RNA-Transkripts bezeichnet, und wenn der Wert $HR_n$ kleiner als 1 ist, er zu $-1/HR_n$ umgewandelt und verwendet wird,

normLogTransValue$_n$ ein Wert ist, der in Bezug zur Expression des n-ten RNA-Transkripts steht, und dieser Wert einen Wert darstellt, dessen Größenordnung sich beruhend auf einem Mittelwert bezüglich aller Werte entsprechender Gene ändert, und die Populationsich auf eine bestimmte Anzahl von Gruppen mit lokal fortgeschrittenem Magenkrebs der Stufe T1N0, Stufe T2N0, Stufe T3N0 oder Stufe T4N0 bezieht, ohne Lymphknotenmetastasen in der TNM-Stufen-Klassifikation, und die bestimmte Anzahl eine beliebige ganze Zahl ist, bei der ein RS-Maximalwert und ein RS-Minimalwert berechenbar sind.

**3.** Verfahren nach Anspruch 1,
wobei das Verfahren zur Vorhersage eines klinischen Ergebnisses nach chirurgischer Resektion von lokal fortgeschrittenem Magenkrebs der Stufe T1N0, Stufe T2N0, Stufe T3N0 oder Stufe T4N0 ohne Lymphknotenmetastasen in der TNM-Stufen-Klassifikation verwendet wird.

**4.** Verfahren nach Anspruch 1,
wobei bei dem Verfahren, wenn RS (%) der biologischen Probe 50% oder mehr beträgt, sie als Hochrisikogruppe eingestuft wird, wenn RS (%) der biologischen Probe 25% oder mehr und weniger als 50% beträgt, sie als Zwischenrisikogruppe eingestuft wird, und wenn RS (%) der biologischen Probe weniger als 25% beträgt, sie als Niedrigrisikogruppe für das Gesamtüberleben (OS) eingestuft wird.

**5.** Computerlesbares Aufzeichnungsmedium mit einem Programm zur Durchführung von Prognosevorhersagen von Magenkrebs, das einen Computer veranlasst, die folgenden Schritte auszuführen:

Beschaffung der Expressionsgrade von RNA-Transkripten von GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 und RRM1, bestimmt in einer von einem Patienten erhaltenen Nukleinsäureprobe;
Berechnung einer Risikobewertung (RS) und eines RS-Prozentsatzes (RS (%)) der Probe auf der Grundlage des Expressionsgrades der RNA, der im obigen Schritt durch die folgenden Gleichung 1 und 2 bestimmt wurde; und
Klassifizierung eines Patienten als Patient einer Hochrisikogruppe, wenn ein festgelegter Wertebereich von RS (%) 50% oder mehr beträgt, als Patient einer Zwischenrisikogruppe, wenn ein festgelegter Wertebereich von RS (%) 25% oder mehr und weniger als 50% beträgt, oder als Patient einer Niedrigrisikogruppe, wenn ein festgelegter Wertebereich von RS (%) weniger als 25% beträgt, für das Gesamtüberleben (OS) ohne Krebsrezidiv nach chirurgischer Resektion von Magenkrebs:

[Gleichung 1]

$$RS = HR_1 * normLogTransValue_1 + HR_2 * normLogTransValue_2 + \ldots + HR_n * normLogTransValue_n$$

[Gleichung 2]

$$RS\,(\%) = 100 \times (RS\text{ der biologischen Probe} - RS\text{-Minimalwert der Population})/(RS\text{-Maximalwert der Population} - RS\text{ Minimalwert der Population})$$

wobei

$HR_n$ einen Risikoquotienten eines n-ten RNA-Transkripts bezeichnet, und wenn der Wert $HR_n$ kleiner als 1 ist, er zu $-1/HR_n$ umgewandelt und verwendet wird,
$normLogTransValue_n$ ein Wert ist, der in Bezug zur Expression des n-ten RNA-Transkripts steht, und dieser Wert einen Wert darstellt, dessen Größenordnung sich beruhend auf einem Mittelwert bezüglich aller Werte entsprechender Gene ändert, und
die Population sich auf eine bestimmte Anzahl von Gruppen mit lokal fortgeschrittenem Magenkrebs der Stufe T1N0, Stufe T2N0, Stufe T3N0 oder Stufe T4N0 bezieht, ohne Lymphknotenmetastasen in der TNM-Stufen-Klassifikation, und die bestimmte Anzahl eine beliebige ganze Zahl ist, bei der ein RS-Maximalwert und ein RS-Minimalwert berechenbar sind.

**Revendications**

**1.** Procédé de prédiction du pronostic pour un sujet chez lequel un cancer gastrique a été diagnostiqué, le procédé comprenant :

une étape de détermination d'un degré d'expression de transcriptions d'ARN de GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 et RRM1 dans un échantillon biologique incluant des cellules cancéreuses prélevé chez le sujet ; et

une étape de calcul d'une cote de risque (RS) et d'un pourcentage de RS (RS (%)) de l'échantillon biologique sur la base du degré d'expression de la transcription d'ARN déterminé à l'étape susmentionnée et de détermination du pronostic selon le RS (%).

2. Le procédé de la revendication 1,
   sachant que le RS et le RS (%) sont calculés par les équations 1 et 2 ci-après :

[Équation 1]

$$RS = HR_1 * normLogTransValue_1 + HR_2 * normLogTransValue_2 + ... + HR_n * normLogTransValue_n$$

[Équation 2]

$$RS\ (\%) = 100 \times (RS\ d'échantillon\ biologique - valeur\ RS\ minimale\ de\ population)/(valeur\ RS\ maximale\ de\ population - valeur\ RS\ minimale\ de\ population)$$

où

$HR_n$ représente un rapport de risque d'une n-ième transcription d'ARN, et lorsque le $HR_n$ est inférieur à 1, il est converti en $-1/HR_n$ et utilisé,

$normLogTransValue_n$ est une valeur relative à l'expression de la n-ième transcription d'ARN, et cette valeur est une valeur dont l'échelle varie en fonction d'une valeur médiane par rapport à toutes les valeurs de gènes correspondants, et

la population se rapporte à un certain nombre de groupes ayant un cancer gastrique localement avancé de stade T1N0, de stade T2N0, de stade T3N0 ou de stade T4N0 ne présentant pas de métastase aux ganglions lymphatiques selon la classification de stade TNM, et le certain nombre est tout entier auquel une valeur maximale et une valeur minimale du RS sont calculables.

3. Le procédé de la revendication 1,
   sachant que le procédé est utilisé pour prédire un résultat clinique après résection chirurgicale d'un cancer gastrique localement avancé de stade T1N0, de stade T2N0, de stade T3N0 ou de stade T4N0 ne présentant pas de métastase aux ganglions lymphatiques selon la classification de stade TNM.

4. Le procédé de la revendication 1,
   sachant que, dans le procédé, lorsque le RS (%) de l'échantillon biologique est de 50 % ou plus, il est déterminé comme groupe à haut risque ; lorsque le RS (%) de l'échantillon biologique est de 25 % ou plus et de moins de 50 %, il est déterminé comme groupe à risque intermédiaire ; et lorsque le RS (%) de l'échantillon biologique est de moins de 25 %, il est déterminé comme groupe à faible risque pour la survie globale (OS).

5. Support d'enregistrement lisible par ordinateur comportant un programme permettant d'exécuter une prédiction du pronostic d'un cancer gastrique, faisant en sorte qu'un ordinateur exécute les étapes suivantes :

   acquisition des degrés d'expression de transcriptions d'ARN de GART, PTN, PCNA, GLI3, SMARCD3, SULT1A3, ILK, FUCA1, PKD1, TOP2A, ABL1, CKS2, FZD1, TIAL1, SGCD, PIGF, CCNB1, CSK, CRYAB, TPM1, RFC4, GUCY1B3, TYMS, FEN1, GNAI1, CSRP1, UNG, AXL, MAP1B, VCL, ITGA5, LIG1, HPRT1, GRB2, HMMR, MCM4, SRF, DMPK, ACP5, CD38, PRIM1, CCNF, GLRB, IFNAR2, HSPA2, CLN3, BUB1, CALM1, CDC2, ATF4 et RRM1 déterminés dans un échantillon d'acide nucléique prélevé chez un sujet ;
   calcul d'une cote de risque (RS) et d'un pourcentage de RS (RS (%)) de l'échantillon sur la base du degré d'expression d'ARN déterminé à l'étape susmentionnée par les équations 1 et 2 ci-après ; et

classification d'un patient comme patient de groupe à haut risque lorsqu'une plage de valeurs définie du RS (%) est de 50 % ou plus, comme patient de groupe à risque intermédiaire lorsqu'une plage de valeurs définie du RS (%) est de 25 % ou plus et de moins de 50 %, ou comme patient de groupe à faible risque lorsqu'une plage de valeurs définie du RS (%) est de moins de 25 % pour la survie globale (OS) sans récidive du cancer après résection chirurgicale de cancers gastriques :

[Équation 1]

$$RS = HR_1 * normLogTransValue_1 + HR_2 * normLogTransValue_2 + ... + HR_n * normLogTransValue_n$$

[Équation 2]

$$RS\,(\%) = 100x(RS\ d'échantillon\ biologique - valeur\ RS\ minimale\ de\ population)/(valeur\ RS\ maximale\ de\ population - valeur\ RS\ minimale\ de\ population)$$

où

$HR_n$ représente un rapport de risque d'une n-ième transcription d'ARN, et lorsque le $HR_n$ est inférieur à 1, il est converti en $-1/HR_n$ et utilisé,
normLogTransValue$_n$ est une valeur relative à l'expression de la n-ième transcription d'ARN, et cette valeur est une valeur dont l'échelle varie en fonction d'une valeur médiane par rapport à toutes les valeurs de gènes correspondants, et
la population se rapporte à un certain nombre de groupes ayant un cancer gastrique localement avancé de stade T1N0, de stade T2N0, de stade T3N0 ou de stade T4N0 ne présentant pas de métastase aux ganglions lymphatiques selon la classification de stade TNM, et le certain nombre est tout entier auquel une valeur maximale et une valeur minimale du RS sont calculables.

【FIG. 1a】

【FIG. 1b】

Good Prognosis Patient
Bad Prognosis Patient

【FIG. 1c】

【FIG. 1d】

【FIG. 2】

EP 2 982 985 B1

【FIG. 3】

31

【FIG. 4a】

A.

Biocarta Pathway(M5_2)

【FIG. 4b】

B.

Biocarta Pathway(M3_3)

【FIG. 5a】

【FIG. 5b】

【FIG. 5c】

【FIG. 5d】

【FIG. 5e】

【FIG. 5f】

【FIG. 6a】

【FIG. 6b】

cell cycle pathway

Fbw7 pathway

fold difference scale

p-value scale

【FIG. 6c】

【FIG. 6d】

【FIG. 6e】

【FIG. 6f】

【FIG. 7a】

A.

Training-CCP

【FIG. 7b】

B.

Training-LDA

【FIG. 7c】

C.

**Training-NC**

【FIG. 7d】

D.

**Test-CCP**

【FIG. 7e】

E.

Test-LDA

【FIG. 7f】

F.

Test-NC

【FIG. 7g】

G.

【FIG. 7h】

H.

【FIG. 7i】

I.

Total-NC

P=0.00012

CL1

CL2

Probability

OS (month)

【FIG. 8a】

A.

Training-CCP

【FIG. 8b】

B. Training-LDA

【FIG. 8c】

C.

Training-NC

【FIG. 8d】

D.

Test-CCP

【FIG. 8e】

E.

Test-LDA

【FIG. 8f】

F.

Test-NC

【FIG. 8g】

G.

**Total-CCP**

P=0.00377

【FIG. 8h】

H.

**Total-LDA**

P=0.00203

【FIG. 8i】

【FIG. 9a】

【FIG. 9b】

B.

Cyclins and Cell Cycle Regulation
Regulation of p27 Phosphorylation during Cell Cycle Progression
Estrogen-responsive protein Efp controls cell cycle and breast tumors growth
Role of BRCA1, BRCA2 and ATR in Cancer Susceptibility
Role of Ran in mitotic spindle regulation
CDK Regulation of DNA Replication
Activation of Src by Protein-tyrosine phosphatase alpha
E2F1 Destruction Pathway
p53 Signaling Pathway
Cell Cycle: G1/S Check Point
Sonic Hedgehog SHH Receptor Ptc1 Regulates cell cycle
cdc25 and chk1 Regulatory Pathway in response to DNA damage
Regulation of cell cycle progression by Plk3
Cell Cycle: G2/M Checkpoint
RB Tumor Suppressor/Checkpoint Signaling in response to DNA damage
Stathmin and breast cancer resistance to antimicrotubule agents
Role of Tob in T-cell activation
Lck and Fyn tyrosine kinases in initiation of TCR Activation
T Cell Receptor and CD3 Complex
T Helper Cell Surface Molecules
T Cytotoxic Cell Surface Molecules
NO2-dependent IL 12 Pathway in NK cells
HIV Induced T Cell Apoptosis
IL 17 Signaling Pathway
Adhesion and Diapedesis of Lymphocytes
IL-7 Signal Transduction
B Cell Receptor Complex
Reelin Signaling Pathway
Small Leucine-rich Proteoglycan SLRP molecules
Classical Complement Pathway
Complement Pathway

【FIG. 10a】

A.

Metastasis
Metabolism
Cell_Cycle
DNA_Damage
transcription factor
Gene Regulation
Signal Transduction
Development
Apoptosis
CL3_3LDA
CL3_3CCP
CL3_3NC
CL5_2LDA
CL5_2CCP
CL5_2NC
DNA_Replication
Immunology
Angiogenesis
Sum of Death Rate
Event

20 patients, 10 death   30 patients, 9 death   27 patients, 5 death   19 patients, 0 death   50 patients, 3 death
(47.6% death rate)   (30% death rate)   (18.5% death rate)   (0% death rate)   (6% death rate)

【FIG. 10b】

B.

## Total Sample

**High Risk Group**
21 patients, 13 death (61.9% death rate)
**Intermediate Risk Group**
70 patients, 14 death (20% death rate)
**Low Risk Group**
67 patients, 5 death (7.46% death rate)

C.

## YG Sample

**High Risk Group**
11 patients, 6 death (54.5% death rate)
**Intermediate Risk Group**
32 patients, 8 death (25% death rate)
**Low Risk Group**
35 patients, 2 death (5.7% death rate)

D.

## MDA Sample

**High Risk Group**
10 patients, 7 death (70% death rate)
**Intermediate Risk Group**
38 patients, 6 death (16% death rate)
**Low Risk Group**
32 patients, 3 death (9% death rate)

【FIG. 10c】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009045115 A1
- KR 20120065959 A

**Non-patent literature cited in the description**

- AJCC cancer staging Manual: stomach. Ann Surg Oncol. 2010, vol. 17, 3077-3079
- **CHO et al.** *Clinical Cancer Research,* 2011, vol. 17 (7), 1850-1857
- **INOUE et al.** *Clinical Cancer Research,* 2002, vol. 8, 3475-3479
- **LEE et al.** *Japanese Journal of Clinical Oncology,* 2003, vol. 33 (4), 173-179
- User Bulletin #2'' for the ABI PRISM 7700 Sequence Detection System. *Applied Biosystems,* 1997